# FASCICULE DE BREVET EUROPEEN

(11) **EP 1 001 806 B1**
(45) Date de publication et mention de la délivrance du brevet: **15.09.2004**
(21) Numéro de dépôt: 98935097.0
(22) Date de dépôt: 03.07.1998
(51) Int. Cl.: A61K 39/00, A61K 39/12, C12N 5/08

(54) **VESICULE CELLULAIRE DENOMMEE 'EXOSOME', PREPARATION ET UTILISATION DANS LA STIMULATION D'UNE REPONSE IMMUNITAIRE**
ZELLULÄRES VESIKEL 'EXOSOME', SEINE HERSTELLUNG UND VERWENDUNG ZUR STIMULIERUNG EINER IMMUNANTWORT
CELLULAR VESICLE CALLED ''EXOSOME'', PREPARATION AND USE THEREOF IN IMMUNE STIMULATION

(30) Priorité: 16.07.1997 FR 9709007; 06.02.1998 FR 9801437
(43) Date de publication de la demande: 24.05.2000
(60) Demande divisionnaire: 04005883.6
(73) Titulaire: INSTITUT NATIONAL DE LA SANTE ET DE LA RECHERCHE MEDICALE (INSERM), 75013 Paris (FR); INSTITUT GUSTAVE ROUSSY, 94805 Villejuif Cédex (FR); CENTRE NATIONAL DE LA RECHERCHE SCIENTIFIQUE (CNRS), 75016 Paris (FR); Institut Curie, 75248 Paris Cedex 05 (FR)
(72) Inventeur: ZITVOGEL, Laurence, F-75014 Paris (FR); RAPOSO, Graça, F-75004 Paris (FR); REGNAULT, Armelle, F-75015 Paris (FR); AMIGORENA, Sebastian, F-75013 Paris (FR)
(74) Mandataire: Becker, Philippe
(86) Numéro de dépôt international: PCT/FR1998/001431
(87) Numéro de publication internationale: WO 1999/003499

(56) Documents cités:
- WO-A-97/05900
- RAPOSO ET AL: "B LYMPHOCYTES SECRETE ANTIGEN-PRESENTING VESICLES" JOURNAL OF EXPERIMENTAL MEDICINE, vol. 183, 1996, pages 1161-1172, XP002060486 cité dans la demande
- AMIGORENA ET AL: "TRANSIENT ACCUMULATION OF NEW CLASS II MHC MOLECULES IN A NOVEL ENDOCYTIC COMPARTMENT IN B LYMPHOCYTES" NATURE, vol. 369, 1994, pages 113-120, XP002085382 cité dans la demande
- TULP ET AL: "ISOLATION AND CHARACTERIZATION OF THE INTRACELLULAR MHC CLASS II COMPARTMENT" NATURE, vol. 369, 1994, pages 120-126, XP002085383
- GRUENBERG ET AL: "CHARACTERIZATION OF THE EARLY ENDOSOME AND PUTATIVE ENDOCYTIC CARRIER VESICLES IN VIVO AND WITH AN ASSAY OF VESICLE FUSION IN VITRO" THE JOURNAL OF CELL BIOLOGY, vol. 108, 1989, pages 1301-1316, XP002085384 cité dans la demande
- TRAMS ET AL: "EXFOLIATION OF MEMBRANE ECTO-ENZYMES IN THE FORM OF MICRO-VESICLES" BIOCHIMICA ET BIOPHYSICA ACTA, vol. 645, 1981, pages 63-70, XP002060487
- BERNHARD ET AL: "GENERATION OF IMMUNOSTIMULATORY DENDRITIC CELLS FROM HUMAN CD34+ HEMATOPOIETIC PROGENITOR CELLS OF THE BONE MARROW AND PERIPHERAL BLOOD" CANCER RESEARCH, vol. 55, 1995, pages 1099-1104, XP002085385 cité dans la demande
- ROMANI ET AL: "PROLIFERATING DENDRITIC CELL PROGENITORS IN HUMAN BLOOD" THE JOURNAL OF EXPERIMENTAL MEDICINE, vol. 180, 1994, pages 83-93, XP002085386 cité dans la demande
- HSU F J ET AL: "VACCINATION OF PATIENTS WITH B-CELL LYMPHOMA USING AUTOLOGOUS ANTIGEN-PULSED DENTRITIC CELLS" NATURE MEDICINE, vol. 2, no. 1, janvier 1996, pages 52-58, XP000605401
- ZITVOGEL L ET AL: "Eradication of established murine tumors using a novel cell-free vaccine: dendritic cell-derived exosomes." NATURE MEDICINE, (1998 MAY) 4 (5) 594-600. JOURNAL CODE: CG5. ISSN: 1078-8956., XP002085387 United States

## Description

L'invention a pour objet un nouveau procédé de sensibilisation de cellules présentatrices d'antigène, de nouveaux moyens pour la mise en oeuvre du procédé, et de nouvelles vésicules membranaires ayant un pouvoir immunogène.

Depuis la démonstration de l'existence de lymphocytes T cytotoxiques CD8+ spécifiques d'antigènes tumoraux présentés dans la contexte des molécules de classe I (Rosenberg et coll., 1996; Boon, 1992), plusieurs laboratoires ont pu montrer que l'immunothérapie anti-tumorale est une stratégie thérapeutique efficace dans les modèles animaux (Pardoll, 1995). Le principe de l'immunothérapie est d'induire une réponse immunitaire efficace contre des antigènes spécifiques de tumeurs. A présent, cela a pu être effectué de différentes façons. Tout d'abord, des cellules tumorales exprimant des molécules de co-stimulation recombinantes et/ou des cytokines immunomodulatrices sont capables de stimuler des réponses anti-tumorales capables d'éradiquer des tumeurs solides *in vivo* (Zitvogel et coll., 1996 [a]). De même, des peptides dérivés d'antigènes tumoraux (ou d'antigènes exogènes exprimés dans les cellules tumorales) injectés sous différentes formes chimiques., y compris l'utilisation de liposomes ou de virus (adénovirus ou poxvirus par exemple) comme vecteurs, sont capables de faire régresser des tumeurs. Finalement, des cellules présentatrices d'antigènes professionnelles, comme les cellules dendritiques sensbilisées avec des peptides dérivés d'antigène tumoraux, re-injectées *in vivo* induisent des réponses anti-tumorales puissantes, ainsi que la régression de tumeurs-solides établies de la souris (Mayordomo et coll., 1995).

Amirogena et al. (cf. : références, page 59) ont décrit l'accumulation, dans le compartiment endocytique des lymphocytes B, de vésicules exprimant des molécules du CMH de classe II.

L'immunothérapie basée sur l'utilisation des cellules dendritiques a pu montrer son efficacité dans les études réalisées chez la souris. De ce fait, cette thérapie a été récemment transposée en clinique. Aux Etats-Unis, des essais sont actuellement en cours afin de démontrer que des cellules dendritiques chargées de peptides tumoraux augmentent de façon significative la fréquence de cellules T cytotoxiques spécifiques (CTL).

Une première limitation à cette approche est la sensibilisation des cellules dendritiques avec des peptides dérivés d'antigènes tumoraux. En effet, dans la majorité des tumeurs, des antigènes spécifiques n'ont pas été identifiés. Des antigènes spécifiques de la tumeur sont seulement connus dans des cas de tumeurs induites par des virus (carcinome du col utérin), dans des cas de mélanome (antigènes du soi, antigènes mutés, antigènes de différentiation) ou dans un petit pourcentage de tumeurs du sein (oncogènes, ou produits de gènes suppresseurs de tumeur ayant subi des mutations). Cependant, l'implication directe de ces peptides ou antigènes tumoraux dans l'élimination des tumeurs chez l'homme reste à démontrer. Des méthodes nouvelles de sensibilisation des cellules présentatrices d'antigènes telles que les cellules dendritiques s'avèrent donc nécessaires. Ces méthodes ont pour but d'induire des réponses anti-tumorales spécifiques dans le contexte de molécules de classe I et de classe II du CMH.

La plupart des méthodes de sensibilisation de cellules dendritiques mises en oeuvre à présent utilisent des peptides correspondant à des épitopes présentés en association avec les molécules de classe I et identifiés dans les cellules tumorales grâce à des clones de CTLs spécifiques de la tumeur. Cependant, ces méthodes ne sont vraisemblablement pas optimales car elles ne tiennent pas compte des épitopes reconnus dans le contexte des molécules de classe II qui sont critiques pour la prolifération des lymphocytes T auxiliaires nécessaires à l'obtention des réponses cytotoxiques optimales. De plus, des épitopes présentés par les cellules tumorales et ceux présentés par les cellules présentatrices d'antigènes (comme par exemple les cellules dendritiques). ne sont probablement pas les mêmes. Finalement, des peptides tumoraux reconnus par les CTL sont seulement disponibles pour un petit pourcentage de patients ayant des molécules de classe I d'haplotype approprié.

La méthode de sensibilisation idéale, de façon à pouvoir être appliquée à n'importe quelle tumeur avec un risque minimal d'immunosélection, ne doit pas être restreinte à un petit nombre d'antigènes tumoraux identifiés. De même, une telle méthode devrait utiliser des antigènes protéiques intacts plutôt que des peptides, afin de permettre à la cellule dendritique de les préparer et de présenter la combinaison adéquate de peptides en association avec les molécules de classe I et de classe II, et cela pour tout individu.

Récemment, Gilboa et collaborateurs (Boczkowsky et coll., 1996) ont pu montrer que des ARN messagers préparés à partir de biopsies de tumeurs chargés dans les cellules dendritiques peuvent avoir un effet anti-tumoral *in vivo.* Cependant les ARN sont très instables et la quantité d'ARN potentiellement intéressant comparé à l'ARN total est vraisemblablement très faible. Zitvogel et coll., (Zitvogel et coll., 1996 [b]) ont montré que des peptides tumoraux préparés à partir d'un éluat acide de tumeurs (éluat peptidique acide : EPA) peuvent être utilisés pour charger des cellules dendritiques. Ces cellules ainsi chargées, une fois injectées, ont la capacité de faire régresser des tumeurs. Cependant, dans le cas de tumeurs n'exprimant pas de molécules de classe I (qui représentent la majorité des tumeurs humaines métastatiques), ou dans le cas de tumeurs qui ne peuvent pas être dissociées en une suspension cellulaire, l'approche utilisant les éluats acides n'est pas très efficace et n'est pas reproductible.

Une deuxième limitation à l'immunothérapie basée sur l'emploi de cellules dendritiques est liée aux changements phénotypiques qui peuvent survenir lorsque ces cellules sont maintenues en culture, ou soumises à différents traitements. Ceci peut en effet conduire à des populations cellulaires peu homogènes et insuffisamment caractérisées pour un usage thérapeutique.

Il existe donc un réel besoin d'améliorer les méthodes de sensibilisation des cellules présentatrices d'antigènes, afin d'augmenter l'efficacité de ces approches et d'élargir leurs applications, ainsi que d'élaborer de nouveaux moyens de vectorisation d'antigènes ou autres molécules.

La présente invention apporte des solutions à ces questions. La présente invention a en effet pour objectif de fournir de nouvelles méthodes de sensibilisation de cellules présentatrices d'antigène, notamment des cellules dendritiques, ainsi que l'identification, l'isolement et la caractérisation des nouvelles vésicules membranaires ayant des propriétés immunogènes remarquables.

L'un des aspects de l'invention est plus particulièrement de proposer un nouveau procédé reproductible de sensibilisation de cellules présentatrices d'antigène par des antigènes tumoraux.

L'un des autres aspects de l'invention est de proposer un nouveau procédé reproductible de sensibilisation de cellules présentatrices d'antigène par des antigènes tumoraux, dans lequel il n'est pas nécessaire que les antigènes tumoraux soient connus.

Un autre aspect est de proposer les moyens permettant d'établir une banque d'antigènes tumoraux.

Un autre aspect de l'invention réside dans des vésicules membranaires, lipidiques, produites par les cellules dendritiques, et douées de propriétés immunogènes, ainsi que leur utilisation pour la production de banques d'antigènes, la sensibilisation de cellules présentatrices d'antigènes ou la vectorisation d'antigènes, notamment dans le cadre d'approches immunothérapeutiques.

A cet égard, un aspect concerne l'utilisation d'une vésicule dérivée de cellules tumorales ayant les caractéristiques suivantes :
- elle est débarrassée de son environnement naturel,
- elle comprend une bicouche lipidique (désignée par "surface") qui entoure une fraction cytosolique,
et éventuellement,
- elle présente sur sa surface des molécules de classe I du complexe majeur d'histocompatibilité (CMH) et/ou de classe Il du complexe majeur d'histocompatibilité (CMH), éventuellement chargées en peptides antigéniques et/ou des molécules d'adhésion et/ou des molécules de co-stimulation lymphocytaire, et/ou,
- elle contient dans sa fraction cytosolique des molécules antigéniques tumorales et/ou des immunomodulateurs et/ou chemo-attracteurs et/ou hormones et/ou des acides nucléiques.

La sécrétion des vésicules par des cellules est un phénomène décrit dans l'art antérieur (réticulocytes, lymphocytes B, macrophages). Ces vésicules sont généralement désignées par le terme générique "exosome" qui reflète leur mécanisme de production par exocytose de vésicules internes. Cependant, le rôle physiologique de ces vésicules n'a pas été vraiment établi. De plus, les caractéristiques structurales, les propriétés et les fonctions de ces vésicules sont variables selon le type cellulaire dont elles sont issues.

De façon inattendue, les inventeurs ont maintenant mis en évidence que les cellules tumorales sont capables de sécréter des vésicules présentant des propriétés immunogènes particulièrement intéressantes. Ces vésicules correspondent généralement à une vésicule interne contenue dans un endosome d'une cellule tumorale et sécrétée par ladite cellule tumorale suite à la fusion de la membrane externe du susdit endosome avec la membrane cytoplasmique de la susdite cellule tumorale. En raison de ce mécanisme de formation, de leur cellule d'origine et de leurs caractéristiques et propriétés fonctionnelles originales, ces vésicules sont désignées dans ce qui suit par le terme "texosome".

L'expression "débarrassée de son environnement naturel" signifie que la vésicule est séparée physiquement de la cellule dont elle est issue, ou encore qu'elle est partiellement isolée ou purifiée. Généralement, la vésicule est donc produite par la cellule par exocytose, puis partiellement isolée ou purifiée de manière à obtenir une composition enrichie. Cette expression peut également signifier que, non seulement la vésicule a été sécrétée par la cellule lors de la fusion des endosomes multivésiculaires avec la membrane plasmique, mais qu'elle n'est plus entourée des éléments solubles qui sont dans le lumen de l'endosome, ou qu'elle est dépourvue de cellules intactes. L'expression "dérivée de cellule tumorale" signifie que la vésicule possède des éléments structuraux d'une cellule tumorale. Cette vésicule est généralement "dérivée" d'une cellule tumorale dans le sens où elle est produite, au moins en partie, puis libérée par une cellule tumorale, à un stade donné de son développement.

Selon un mode de réalisation avantageux, les texosomes présentent des molécules du CMH chargées en peptides antigéniques et/ou expriment des molécules d'adhésion et/ou expriment des molécules de co-stimulation lymphocytaire, mais sont dépourvus, dans leur fraction cytosolique, de molécules antigéniques tumorales et d'immunomodulateurs et d'acides nucléiques.

Selon un autre mode de réalisation avantageux, les texosomes sont tels que les molécules du CMH sont "vides", c'est-à-dire non chargées de peptides antigéniques et les texosomes comprennent, dans leur fraction cytosolique, des molécules antigéniques tumorales, des immunomodulateurs et/ou des acides nucléiques. Des texosomes ayant des molécules du CMH vides peuvent être obtenues soit à partir de cellules, tumorales présentant par exemple une déficience pour le transporteur de peptides (TAP), soit par lavage de texosomes ou de cellules tumorales, afin d'éluer les peptide associés aux molécules du CMH.

Selon un mode de réalisation avantageux de l'invention, les texosomes sont tels que les molécules du CMH sont chargées en peptides antigéniques et/ou expriment des molécules d'adhésion et/ou en molécules de co-stimulation lymphocytaire et les texosomes contiennent dans leur fraction cytosolique des molécules antigéniques tumorales, des immunomodulateurs et/ou des acides nucléiques.

Le terme "cellules tumorales" englobe de manière générale toute cellule provenant d'une tumeur, par exemple une tumeur solide ou liquide, ainsi que les cellules transformées ou immortalisées in vitro. Il s'agit de préférence d'une tumeur solide, ascitique ou hématopoïétique.

On peut citer, à titre d'exemple, des cellules de cancer de type mélanome malin (provenant de lignées primaires établies *"ex vivo"* ou bien de cellules dissociées provenant de la pièce opératoire) qui expriment à leur surface des peptides comme MART-1/Melan-A, dans le contexte CMH - classe 1, HLA-A 02-01, et contenant l'antigène protéique MART-1.

On peut également citer des cellules provenant de cancer du rein (adénocarcinome à cellules claires) ou de leucémies dont les cellules expriment des produits spécifiques de translocation.

Ainsi, les peptides antigéniques susceptibles de charger les molécules du CMH proviennent par exemple des antigènes suivants : ceux provenant de mélanomes tels que : MART-1, tyrosinase, MAGE-1/2/3. P53 (dans différentes tumeurs) ou Her2/Neu, PSA, CEA ou encore PSMA. D'autres antigènes tumoraux sont cités par exemple dans l'article de Rosenberg (Immunology Today 18 (1997) 175).

Plus généralement, on peut citer des produits de fusion/translocation, d'oncogènes ou d'anti-oncogènes, ou bien des antigènes de différenciation ou des peptides du soi ou peptides mutés.

Par molécules de co-stimulation lymphocytaire, on désigne par exemple des molécules qui donnent aux lymphocytes T des signaux complémentaires à ceux donnés lors de l'interaction des complexes Molécule de classe I et II-peptide avec le récepteur des cellules T.

On peut citer, à titre d'exemple :

CD80, CD86, ICAM, LFA, CD40, certains membres de la famille TNF R et des molécules d'adhésion ou de chemo attraction (permettant le contact entre la cellule professionnelle présentatrice d'antigène et les lymphocytes effecteurs, ou le transport intracellulaire/localisation spécifique ("trafficking/homing") d'autres cellules au site vaccinal ou inflammatoire.

Les molécules antigéniques tumorales contenues dans le cytosol ou présentées par les texosomes proviennent de protéines exprimées de façon sélective et/ou abondante par les cellules tumorales.

Les immunomodulateurs qui peuvent être présents dans le cytosol des texosomes sont par exemple:
- TNF-α, ou
- Interleukine 1, ou
- Interleukine 15, ou
- C-CR (chemokines).

Les acides nucléiques susceptibles d'être présents dans le cytosol des texosomes proviennent de la cellule tumorale elle-même. Ces acides nucléiques se trouvent dans le cytosol des texosomes en conséquence directe de leur mécanisme de formation. Il peut aussi s'agir d'acides nucléiques hétérologues.

Des caractéristiques plus particulières des texosomes sont les suivantes :
- ce sont de petites vésicules membranaires de 60 à 100 nm environ, le plus souvent de 60 à 90 nm environ, notamment de 60 à 80 nm, sécrétées par les cellules tumorales,
- ils possèdent des molécules normalement présentes dans les endosomes,
- ils contiennent des antigènes tumoraux, comme par exemple MART-1 dans le cas de cellules de mélanome,
- ils sont dépourvus de cellules mortes, et/ou débris cellulaires,
- ils sont dépourvus de contaminants tels que contaminants membranaires, réticulum endoplasmique, appareil de Golgi, mitochondries ou constituants de noyaux,
- ils portent à leur membrane des molécules de classe I / II fonctionnelles chargées de peptides antigéniques tumoraux,
- ils peuvent stimuler in vitro la prolifération de lymphocytes T spécifiques,
- ils peuvent sensibiliser *in vivo* et *in vitro* des cellules dendritiques capables ensuite d'activer des cellules T spécifiques de la tumeur,
- ils présentent la capacité lorsqu'ils sont inoculés *in vivo,* notamment en intradermique, de faire régresser des tumeurs solides établies,
- ils portent des molécules de costimulation lymphocytaire telles que CD40 et CD80, et/ou,
- ils contiennent la protéine ("heat-shock") HSP70,
- ils sont dépourvus de la protéine gp96.
- ils contiennent des interleukines, ou des chemo-attractants ou des immunomodulateurs.

Une autre caractéristique intéressante des texosomes est qu'ils contiennent de la phosphatidylsérine dans leur feuillet externe. La phosphatidylsérine (PS) est un des composants majeurs des membranes cellulaires, normalement présent très majoritairement dans le feuillet interne des bicouches lipidiques. Dans certaines circonstances, comme les étapes précoces de l'apoptose, la PS est re-distribuée vers le feuillet externe. La présence de PS dans le feuillet externe de la membrane cytoplasmique des cellules apoptotiques constitue un signal de reconnaissance par les macrophages. Afin de déterminer si la PS est exposée à la surface des texosomes, des préparations d'exosomes purifiés à partir de sumageants des cellules de mélanome humain FON ont été analysées par la méthode décrite par Aupeix et al. (J. Clin. Invest. 99: 1546-1554, 1997). La teneur en phosphatidylsérine dans le feuillet externe des échantillons FON (contenant 390 microg/ml de protéines) est de 460 nM de PS. Les exosomes contiennent donc des quantités importantes de PS dans leur feuillet externe.

Des tests permettant de vérifier que les texosomes possèdent des molécules normalement présentes dans les endosomes consistent en la microscopie électronique et l'immunoempreinte (Western Blot). Ces tests permettent de montrer que les texosomes expriment le récepteur de la transférine, des molécules LAMP ("lysozome associated membrane protein" : protéine membranaire associée au lysozome), des molécules de classe I/II, des antigènes tumoraux.

Un test permettant de vérifier que les texosomes sont dépourvus de contaminants est la microscopie électronique et immunoempreinte avec des anticorps anti-calnexine qui est présente dans le réticulum endoplasmique.

Un test permettant de vérifier que les texosomes portent à leur membrane des molécules de classe I/II fonctionnelles chargées de peptides antigéniques tumoraux consiste en une présentation antigénique à des lymphocytes T spécifiques des antigènes de la tumeur concernée (tests de prolifération de clones T spécifiques d'antigènes et CMH classe I restreints).

On peut également utiliser un test de sécrétion de cytokines (IFNγ, GM-CSF, TNFβ) par les clones T sus-cités.

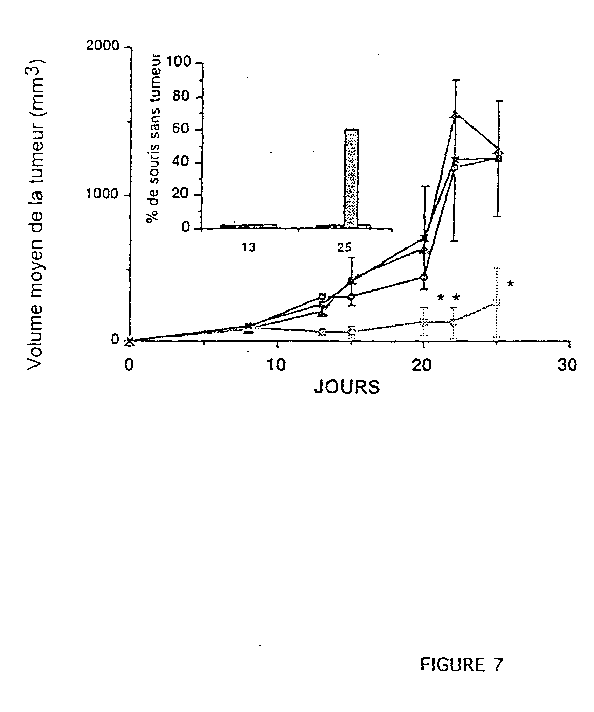

Un test permettant de vérifier qu'il y a sensibilisation *in vivo* et *in vitro* des cellules dendritiques capables d'activer des cellules T spécifiques de la tumeur est donné par la Figure 7 (test de prolifération et/ou sécrétion de cytokines par les clones T spécifiques d'antigènes, par la méthode de sensibilisation croisée ("cross-priming") : texosomes d'une tumeur MART-1+, HLA-A2- chargés sur une cellule dendritique MART-1-, HLA-A2+).

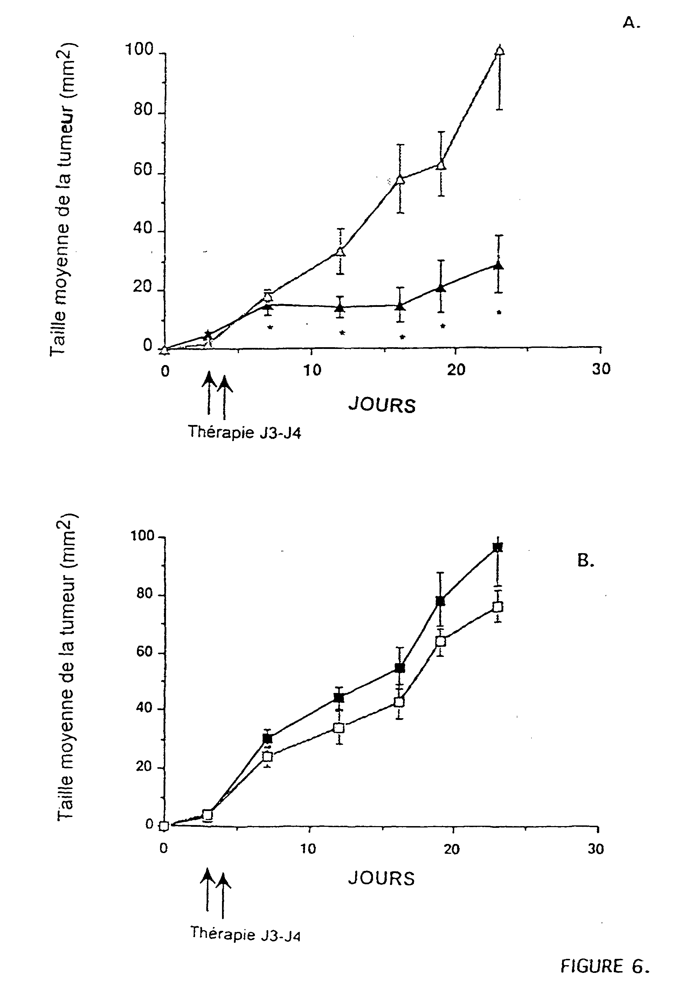

Un test permettant de vérifier que les texosomes présentent la capacité, lorsqu'ils sont inoculés, notamment en intradermique, de faire régresser des tumeurs solides établies est donné à la figure 6.

A titre d'exemple, on pratique une injection de 10 à 40 µg de texosomes de tumeur en intradermique du côté homolatéral à la tumeur établie depuis 3 à 10 jours ; on observe l'animal porteur de la tumeur et la disparition progressive de la tumeur établie en 7 à 10 jours (chez les rongeurs de type souris).

Un texosome avantageux est constitué par un texosome tel que défini ci-dessus et présentant sur sa surface des molécules de classe I et/ou de classe II du CMH, éventuellement chargée en peptides antigéniques et contenant dans sa fraction cytosolique des molécules antigéniques tumorales. Plus particulièrement, le texosome préféré comprend également une ou plusieurs molécules de co-stimulation lymphocytaire et/ou la protéine HSP70. Dans un mode particulier de réalisation, le texosome est dépourvu de la protéine gp96.

Selon un mode de réalisation avantageux, l'invention utilise un texosome tel que défini ci-dessus,
- exprimant à sa surface des molécules de classe et/ou de classe II du complexe majeur d'histocompatibilité (CMH), et/ou des antigènes caractéristiques de tumeurs et/ou des molécules de co-stimulation lymphocytaire/adhésion et/ou des immunomodulateurs, et/ou chemo-attractants, exogènes par rapport à la cellule tumorale dont dérive l'exosome, ou
- contenant des antigènes tumoraux et/ou des immunomodulateurs et/ou des acides nucléiques ou des agents cytotoxiques ou des hormones exogènes par rapport à la cellule tumorale dont dérive l'exosome.

Un procédé de préparation de texosomes tels que définis ci-dessus est décrit. Ce procédé comprend avantageusement une étape de mise à disposition d'un échantillon biologique et une étape d'isolement de texosomes à partir dudit échantillon.

L'échantillon biologique est avantageusement constitué de fractions membranaires, de sumageants de culture ou de lysats de cellules tumorales, ou bien de suspensions tumorales fraîches.

L'échantillon biologique peut provenir de pièces tumorales opératoires après excision chirurgicale (1er cas) ou bien d'organes porteurs de tumeur (organe excisé chirurgicalement) (2eme cas), que l'on traite par dissociation mécanique (1 er cas) ou par perfusion prolongée (2eme cas).

La suspension cellulaire finale est traitée de la même manière que les sumageants de culture.

Il peut aussi s'agir de cellules traitées par congélation/décongélation en plusieurs cycles successifs.

Selon un mode de réalisation avantageux, l'échantillon biologique utilisé est :
- un prélèvement de sang efférent de la veine de l'organe tumoral isolé, ou
- un prélèvement de sérum ou de plasma du sang circulant d'un patient, ou
- le produit de drainage (sérum physiologique contenant éventuellement de la dexaméthasone, ou agent cytotoxique stimulant l'exocytose des texosomes) d'un organe excisé chirurgicalement et traité *ex vivo* par circuit isolé-perfusé pour le drainage de la tumeur qu'il porte, ou encore
- le surnageant d'un expiant tumoral dissocié *in vitro.*

Le prélèvement de sang efférent de l'organe tumoral isolé correspond à 20 à 50 ml de sang de la veine principale efférente de l'organe tumoral, prélevé avant l'intervention d'ablation chirurgicale.

Le produit de drainage d'un organe excisé chirurgicalement et traité ex *vivo* par circuit isolé-perfusé à lieu de la façon suivante.

Dans le cas d'organe présentant une artère afférente et une veine efférente, on caractérise l'artère par une tubulure plastique branchée à une poche proclive contenant du sérum physiologique avec éventuellement d'autres agents. On draine l'organe et le liquide ressort par une autre tubulure cathétérisant la veine, en déclive (par exemple dans le cas du cancer du rein, ou d'un glioblastome cérébral).

La dexaméthasone, contenue éventuellement dans le produit de drainage, a pour but d'augmenter le stress cellulaire et l'exocystose des texosomes hors de la cellule tumorale.

Le surnageant d'un explant tumoral dissocié *in vitro* est obtenu de la façon suivante :
- on procède à la dissociation mécanique de la tumeur conduisant à une suspension unicellulaire contenant des cellules tumorales et des cellules du stroma tumoral et des cellules du système immunitaire ; cette suspension peut être irradiée et récupérée pour les ultracentrifugations différentielles.

Comme indiqué ci-dessus, l'échantillon biologique peut être traité par un ou plusieurs agents stimulant la production de texosomes. Ce traitement peut comprendre l'addition d'agents stéroïdes (déxaméthazone par exemple), d'agents pharmacologiques (par exemple des agents cytotoxiques tels que taxanes, cis-platine, etc), d'agents susceptibles d'augmenter la quantité d'endosomes multivésiculaires et/ou une irradiation de l'échantillon.

S'agissant de l'irradiation, elle doit être suffisante pour provoquer l'action cytostatique des cellules tumorales. L'irradiation des cellules tumorales peut être faite avant la mise en culture des cellules, ou pendant ou après la mise en culture des cellules tumorales. Par ailleurs, il convient d'irradier quand les cellules tumorales sont vivantes, c'est-à-dire :
- soit sur l'organe excisé porteur de tumeur avant la perfusion,
- soit sur les cellules en culture,
- soit sur la suspension cellulaire dissociée mécaniquement ; mais, dans tous les cas, avant souffrance cellulaire tumorale pour cause d'hypoxie/nécrose vasculaire/déshydratation.

S'agissant du traitement à l'aide de stéroïdes, il permet de provoquer une activation cellulaire conduisant à l'exocytose des texosomes.

S'agissant du traitement à l'aide d'agents pharmacologiques, il permet de :
- modifier le cytosquelette et réarranger les compartiments intracellulaires pour dérégler les phénomènes d'internalisation et d'exocytose,
- dépolymériser les microtubules.

S'agissant du traitement avec un agent susceptible d'augmenter la quantité d'endosomes multivésiculaires, il a lieu pendant la mise en culture des cellules, comme agent on peut citer le nocodazole, (drogue permettant de dépolymériser les microtubules), la bafilomycine (drogue inhibant les Atpases vacuolaires)("Bafilomycins: A class of inhibitors of membrane ATPases from microorganisms, animal cells, and plant cells" (1988) Proc. Natl. Acad. Sci. USA 85 : 7972-7976),

Un procédé avantageux de préparation de texosomes est effectué :
a) soit à partir de cultures de cellules tumorales, et comprend:
   - une irradiation, à une intensité suffisante pour provoquer l'action cytostatique des cellules tumorales et ne dépassant pas 15.000 rads avantageusement à environ 10.000 rads, des cellules tumorales avant, pendant ou après leur mise en culture, ou
   - un traitement, pendant la culture, des cellules tumorales, à l'aide de stéroïdes, par exemple dexaméthasone, ou d'agents cytotoxiques, par exemple 5-fluorouracile (5 Fu) ou cis-platine, docetaxel, anthracycline, poison du fuseau, antipyrimidique, ou d'interleukine par exemple IL 10, IL2, IL 15, GM-CSF, ou,
   - un traitement avec un agent susceptible d'augmenter la quantité d'endosomes multivésiculaires, par exemple le nocodazole (Gruenberg J. et al., (1989) "Characterization of the Earty Endosome and Putative Endocytic Carrier Vesicles *In vivo* and with an Assay of Vesicle Fusion *In vitro"* The Journal of Cell Biology 108: 1301-1316) et donc d'augmenter la production de texosomes,
b) soit à partir d'un prélèvement de sérum physiologique drainant un organe excisé chirurgicalement et traité *ex vivo* par circuit isolé-perfusé pour le drainage de la tumeur qu'il porte, ou
c) soit à partir du surnageant d'un explant tumoral dissocié *in vitro* et comprenant :
   - un traitement à l'aide de stéroïdes, par exemple dexamethasone, ou d'agents cytotoxiques, par exemple 5-fluorouracile (5-Fu); cis-platine, taxanes, ou d'interleukine par exempte IL-10; IL-2, GM-CSF.

L'étape d'isolement des texosomes peut être réalisée selon différentes techniques telles que la centrifugation, la chromatographie, l'électrophorèse, la nanofiltration etc. il s'agit par exemple d'une centrifugation différentielle de fractions membranaires de sumageants de culture ou de lysats de cellules tumorales ou de suspensions tumorales fraîches et de récupération de la ou des fraction(s) contenant lesdits exosomes (Raposo et al., J. Exp. Med. 1996, 183 : 1161-1172). Dans ce mode particulier de mise en oeuvre, la fraction membranaire de surnageants est celle obtenue après ultracentrifugation à 100.000 g. Il peut s'agir avantageusement d'une électrophorèse en phase fluide, qui permet la séparation de matériels biologiques d'après leur charge. Les exemples qui suivent montrent que cette technique peut être avantageusement utilisée pour l'isolement de texosomes avec de bons rendements. Cette technique est par ailleurs particulièrement avantageuse sur le plan industriel.

Un procédé de préparation de texosomes tels que défini ci-dessus, comprenant en outre :
- soit la modification génétique des cellules tumorales par des gènes exogènes codant pour des molécules de classe I et/ou de classe II du complexe majeur d'histocompatibilité (CMH), et/ou des gènes codant pour des antigènes caractéristiques de tumeurs et/ou des gènes codant pour des molécules de co-stimulation/adhésion ou des chemokines attractantes, les produits de ces gènes exogènes pouvant se trouver exprimés à la surface des texosomes et/ou être séquestrés à l'intérieur des texosomes,
- soit la modification *in vitro* des texosomes produits par les cellules tumorales, telle que l'introduction (par électroporation, par fusion avec un liposome synthétique, par virus recombinant ou par méthode chimique), de protéines ou d'acides nucléiques ou médicaments pharmaceutiquement définis dans et/ou avec les texosomes.

Les texosomes des cellules tumorales transfectées comme indiqué ci-dessus sont recueillis et utilisés comme vaccins tumoraux.

Les texosomes modifiés *in vitro* comme indiqué ci-dessus sont destinés à délivrer la matériel exogène à une cellule cible *in vitro* ou *in vivo.*

S'agissant de la fusion avec un liposome synthétique, ce procédé est réalisé par exemple comme indiqué dans Nabel et al. (1996) ou dans Walker et al. (1997, Nature 387, pages 61 et suivantes).

L'invention concerne également une vésicule membranaire débarrassée de son environnement naturel, sécrétée par une cellule dendritique et comportant des molecules du complexe majeur d'histocompatibilité de classe I et des molécules du complexe majeur d'histocompatibilite de classe II. d'antigène chargées en texosomes tels que définis ci-dessus.

Pour obtenir ces vésicules membranaires définies ci-dessus, on peut avoir recours à un procédé comprenant :
- une étape de préparation d'un texosome tel que défini ci-dessus,
- une étape d'incubation d'un texosome avec des cellules présentatrices d'antigène,
- une étape de centrifugation différentielle de fractions membranaires de surnageants de culture ou de lysats des susdites cellules présentatrices d'antigènes, chargées en texosomes et
- une étape de récupération de la fraction contenant les susdites vésicules membranaires.

A cet égard, l'invention concerne des vésicules membranaires produites par des cellules dendritiques. De manière inattendue, les inventeurs ont en effet mis en évidence que les cellules dendritiques étaient capables de produire des vésicules membranaires ayant des propriétés immunogènes particulièrement avantageuses. De telles vésicules ont en particulier été visualisées, isolées et caractérisées à partir de surnageants de culture de cellules dendritiques, notamment de cellules dendritiques humaines immatures. Contrairement aux vésicules décrites jusqu'à présent, ces vésicules sont tout à fait avantageuses dans la mesure où elles présentent, de manière importante et simultanée, des molécules des complexes majeurs d'histocompatibilité des classes ) I et II. Ces vésicules membranaires comprennent une bicouche lipidique entourant une fraction cytosolique, et sont désignées dans ce qui suit par le terme "dexosome" en raison de leur origine et de leurs propriétés biochimiques et biologiques originales. Ces vésicules possèdent en effet des propriétés immunogènes remarquables puisqu'elles sont capables de stimuler la production et l'activité de lymphocytes T cytotoxiques, à la fois in vitro et in vivo, et qu'elles permettent de supprimer in vivo la croissance de tumeurs établies, d'une manière dépendante des lymphocytes T et restreinte au type de MHC. Les dexosomes constituent donc des principes actifs particulièrement adaptés pour des approches non-cellulaires d'immunothérapie.

Des vésicules membranaires au sens de l'invention sont donc des vésicules susceptibles d'être produites par des cellules dendritiques, et comportant une ou plusieurs molécules de complexe majeur d'histocompatibilité de classe I et une ou plusieurs molécules de complexe majeur d'histocompatibilité de classe II.

Les dexosomes comportent avantageusement des molécules de co-stimulation lymphocytaires, et en particulier des molécules CD63 et/ou CD82 et/ou CD86, de préférence au moins CD86. Les études présentées dans les exemples montrent en effet que les dexosomes sont fortement marqués par des anticorps dirigés spécifiquement contre ces molécules de co-stimulation.

Par ailleurs, les analyses en microscopie électronique montrent que les dexosomes sont homogènes et possèdent un diamètre compris entre 60 et 100 nm environ, le plus souvent entre 60 et 90 nm environ.

Une variante particulièrement préférée de l'invention est donc représentée par un dexosome ayant un diamètre compris entre 60 et 90 nm environ, obtenu à partir d'une cellule dendritique, et comportant:
- une ou plusieurs molécules du complexe majeur d'histocompatibilité de classe I,
- une ou plusieurs molécules du complexe majeur d'histocompatibilité de classe II,
- une ou plusieurs molécules CD63,
- une ou plusieurs molécules CD86; et,
- une ou plusieurs molécules CD82.

Dans un mode de réalisation particulier de l'invention, les dexosomes comportent en outre un ou plusieurs peptides antigéniques et/ou sont obtenus à partir de cellules dendritiques immatures.

Toujours selon un mode de réalisation particulier, les dexosomes sont dépourvus des marqueurs H2-M, chaîne li, et calnexine (un marqueur spécifique du réticulum endoplasmique).

D'autre part, toujours selon un mode de réalisation avantageux, les dexosomes de l'invention comportent en outre de la phosphatidylsérine (PS) dans leur feuillet externe. Ainsi, des préparations d'exosomes purifiés à partir de sumageants de cellules dendritiques dérivées de la moelle osseuse ont été analysées par la méthode décrite par Aupeix et al. (J. Clin. Invest. 99: 1546-1554, 1997). La teneur en phosphatidylsérine dans le feuillet externe des échantillons BMDC (contenant 35 microg/ml de protéines), elle est de 80 nM de PS. Les dexosomes contiennent donc des quantités importantes de PS dans leur feuillet externe.

Lés dexosomes peuvent être préparés selon une méthodologie comprenant une première étape d'obtention de cellules dendritiques ou de culture cellulaire comprenant des cellules dendritiques, une deuxième étape, facultative, au cours de laquelle les cellules peuvent être sensibilisées à des antigènes d'intérêt, et une troisième étape comprenant la production de dexosomes à partir de ces cultures cellulaires. Ces différentes étapes peuvent être réalisées avantageusement selon les méthodologies décrites ci-après.

### Préparation des cellules dendritiques

La première étape du procédé comprend la mise à disposition d'une (de) culture(s) de cellules dendritiques. Il peut s'agir de cultures de cellules enrichies en cellules dendritiques, voire de cultures cellulaires comprenant essentiellement des cellules dendritiques. Avantageusement, il s'agit bien évidemment de cellules dendritiques humaines.

La préparation de cellules dendritiques a été bien documentée dans la littérature. Ainsi, il est connu que ces cellules peuvent être obtenues à partir de cellules souches du système immunitaire ou à partir de précurseurs monocytes, ou encore isolées directement sous forme différenciée (Revue par Hart, Blood 90 (1997) 3245).

L'obtention de cellules dendritiques à partir de cellules souches est illustrée par exemple par Inaba et al. (J. Exp. Med. 176 (1992) 1693), Caux et al. (Nature 360 (1992) 258) ou Bernhard et al. (Cancer Res. 55 (1995) 1099). Ces travaux montrent notamment que des cellules dendritiques peuvent être produites par culture de moelle osseuse en présence de Facteur de Stimulation des Colonies de Granocytes-Macrophages (GM-CSF) ou, plus précisément, à partir de cellules souches hématopoïétiques (CD34+) par culture en présence d'une combinaison de cytokines (GM-CSF + TNFα).

L'obtention de cellules dendritiques à partir de précurseurs monocytes est illustrée par exemple par Romani et al. (J. Exp. Med. 180 (1994) 83), Sallusto et al. (J. Exp. Med. 179 (1994) 1109), Inaba. et al. (J. Exp. Med. 175 (1992) 1157) ou encore Jansen et al. (J. Exp. Med. 170 (1989) 577). Ces méthodologies reposent essentiellement sur le prélèvement de cellules mononuclées dans le sang et la mise en culture en présence de différentes combinaisons de cytokines: Une méthode particulière consiste à traiter les précurseur monocytes du sang en présence de combinaisons de cytokines telles que Interleukine-4 + GM-CSF ou Interleukine-13 + GM-CSF par exemple. Cette technique est également illustrée par Mayordomo et al., 1995. Par ailleurs, il est également possible de traiter les précurseurs monocytes par des agents pharmacologiques de différenciation cellulaire, tels que des activateurs de canaux calciques.

Une autre approche pour l'obtention de cellules dendritique consiste à isoler, à partir d'échantillons biologiques, des cellules dendritiques déjà différenciées. Cette approche a été décrite par exemple par Hsu et al. (Nature Medicine 2 (1996) 52). La méthodologie décrite par cette équipe consiste essentiellement à récolter des échantillons de sang périphérique et à les traiter par différents gradients et centrifugations de manière à en extraire les cellules dendritiques.

La méthodologie repose sur la production de cellules dendritiques à partir de précurseurs monocytes ou de moelle osseuse. Ces méthodologies sont illustrées dans les exemples. Plus particulièrement, on préfère utiliser dans le cadre de la présente invention des cellules dendritiques obtenues par traitement de précurseurs monocytes (contenus dans le sang ou la moelle) en présence d'une combinaison GM-CSF+IL-4 ou GM-CSF+IL-13.

Par ailleurs, pour la mise en oeuvre de la présente invention, il est tout particulièrement avantageux d'utiliser une population de cellules dendritiques comprenant des cellules dendritiques immatures. Avantageusement, on utilise une population de cellules dendritiques composée principalement (i.e., au moins 60%, de préférence 70%) de cellules dendritiques immatures. L'état immature des cellules dendritiques correspond à un stade précoce de leur développement, auquel elles présentent une forte activité endocytique et expriment des niveaux faibles de molécules de classe I et II du CMH et de molécules de co-stimulation lymphocytaire à leur surface. De manière surprenante, les inventeurs ont en effet trouvé que seules les cellules dendritiques immatures étaient capables de produire des vésicules membranaires en quantité significative. Cette découverte est d'autant plus surprenante que les cellules dendritiques au stade immatures sont connues pour leur faible capacité à stimuler les lymphocytes T, et donc pour leur faible activité biologique (Cella, Nature London, 388 (1997) 782).

La première étape du procédé de l'invention peut donc comprendre avantageusement la préparation d'une population de cellules dendritiques comprenant des cellules dendritiques immatures, notamment à partir de précurseurs monocytes, plus particulièrement par traitement avec une combinaison de cytokines telle que GM-CSF+IL-4 ou GM-CSF+IL-13.

Par ailleurs, il est également possible d'utiliser dans le cadre de la présente invention des populations de cellules dendritiques immortalisées. Il peut s'agir de lignées de cellules dendritiques immortalisées (lignée D1 utilisée dans les exemples, ou tout autre lignée produite par exemple par introduction de l'oncogène myc dans les cellules dendritiques). Il peut également s'agir de cellules dendritiques préparées puis immortalisées in vitro. L'intérêt de cellules dendritiques immortalisées réside dans la constitution de banques de cellules sensibilisées à des groupes d'antigènes donnés, utilisables industriellement pour préparer des dexosomes susceptibles d'être administrés à des familles entières de patients.

Lorsque les cellules dendritiques sont préparées, elles peuvent être maintenues en culture, purifiées d'avantage, stockées ou utilisées directement dans les étapes suivantes du procédé.

### Sensibilisation des cellules dendritiques

Les dexosomes de l'invention peuvent être préparés à partir de cellules dendritiques non chargées en antigènes, c'est-à-dire ne comportant pas d'antigènes déterminés dans leurs membranes ou leur cytosol. De tels dexosomes sont alors désignés "naïfs" ou "vierges".

Selon un mode préféré de mise en oeuvre, les dexosomes de l'invention sont toutefois préparés à partir de cellules dendritiques sensibilisées à un antigène ou à un groupe d'antigènes. Dans ce mode de réalisation, les dexosomes sont en effet eux-mêmes porteurs dudit ou desdits antigènes et sont ainsi capables d'induire une réponse à l'encontre de ceux-ci.

Différentes techniques peuvent être utilisées pour sensibiliser les cellules dendritiques à des antigènes. Ces techniques ont été évoquées plus haut et comprennent notamment :
- la mise en contact des cellules dendritiques avec des peptides antigéniques ("peptide pulsing"). Cette approche consiste à incuber les cellules dendritiques, pendant un temps variable (généralement de 30 minutes à 5 heures environ) avec un ou plusieurs peptides antigéniques, c'est-à-dire avec un peptide issu d'un antigène, tel qu'il pourrait résulter du traitement dudit antigène par une cellule présentatrice de l'antigène. Ce type d'approche a été décrit par exemple pour des peptides antigéniques du virus HIV, de l'Influenza ou de HPV ou pour des peptides dérivés des antigènes Mut1, Mart, Her2 ou Neu par exemple (Macatonia et al., J. Exp. Med. 169 (1989) 1255 ; Takahashi et al., Int. Immunol. 5 (1993) 849 ; Porgador and Gilboa, J. Exp. Med. 182 (1995) 255 ; Ossevoort et al., J. Immunother. 18 (1995) 86 ; Mayordomo et al., précitée ; Mehta-Damani et al., J. Immunol. (1994) 996). Il est également possible d'incuber les cellules dendritiques avec un éluat peptidique acide d'une cellule tumorale selon la méthodologie décrite par Zitvogel et al. (1996, précitée).
- la mise en contact des cellules dendritiques avec un ou plusieurs antigènes ("antigen pulsing"). Cette approche consiste à incuber les cellules dendritiques non pas avec un ou plusieurs peptides antigéniques, mais avec le ou les antigènes intacts. L'intérêt de cette technique réside dans le fait que l'antigène va être transformé en peptides antigéniques par les mécanismes naturels de la cellule dendritique, de sorte que les peptides antigéniques résultant et présentés par la cellule dendritique devraient procurer une meilleure immunogénicité. Cette approche a été illustrée par exemple par inaba et al. (J. Exp. Med. 172 (1990) 631) ou par Hsu et al., (Nature Medicine 2 (1996) 52).
- la mise en contact des cellules dendritiques avec un ou plusieurs complexes protéiques antigéniques. Cette approche est similaire à la précédente mais peut permettre d'améliorer l'efficacité de transformation et/ou de présentation de l'antigène. En particulier, l'antigène peut être utilisé sous forme soluble ou complexée à des éléments de ciblage, permettant notamment de cibler des récepteurs membranaires comme les récepteurs du mannose ou les récepteurs d'immunoglobulines (Rfc). Il est également possible de rendre l'antigène particulaire de manière à améliorer sa pénétration ou encore sa phagocytose par les cellules.
- la mise en contact des cellules dendritiques avec des cellules ou membranes de cellules exprimant des antigènes ou peptides antigéniques. Cette technique repose sur le transfert direct d'antigènes ou peptides antigéniques par fusion de cellules ou de membranes cellulaires. Cette approche a été illustrée par exemple par la fusion entre des cellules dendritiques et des membranes de cellules tumorales (Zou et al., Cancer Immunol. Immunother. 15 (1992) 1).
- la mise en contact des cellules dendritiques avec des vésicules membranaires contenant des antigènes ou peptides antigéniques (notamment des exosomes de cellules tumorales tels que décrits ci-avant). Cette approche de sensibilisation des cellules dendritiques utilisant des exosomes, telle que mise en évidence dans la présente invention, est particulièrement avantageuse dans la mesure où elle ne nécessite pas la connaissance des antigènes particuliers et où les peptides antigéniques chargés sont dans une conformation native. Cette technologie est illustrée dans les exemples.
- la mise en contact des cellules dendritiques avec des liposomes contenant des antigènes ou peptides antigéniques (Nair et al., J. Exp. Med. 175 (1992) 609).
- la mise en contact des cellules dendritiques avec des ARNs codant pour des antigènes ou peptides antigéniques, (voir Boczkowsky et al., 1996, précité).
- la mise en contact des cellules dendritiques avec des ADNs codant pour des antigènes ou peptides antigéniques (éventuellement incorporés dans des vecteurs de type plasmidique, viral ou chimique). Ainsi, un mode de sensibilisation des cellules dendritiques consiste par exemple à infecter les cellules dendritiques avec un virus contre lequel une protection est recherchée. Ceci a été décrit par exemple pour le virus de l'influenza (Bhardwaj et al., J. Clin. Invest. 94 (1994) 797 ; Macatonia et al., précitée). Une autre approche consiste à délivrer, au moyen d'un virus ou d'autres vecteurs de transfert d'acides nucléiques, un ADN codant pour le ou les antigènes ou peptides antigéniques d'intérêt. Une telle approche a été illustrée par exemple par Arthur et al. (Cancer Gene Therapy, 1995) ou par Alijagie et al. (Eur. J. Immunol. 25 (1995) 3100). Certains virus tels les adénovirus, les AAV ou les rétrovirus semblent pouvoir être utilisés à cet effet, pour délivrer un acide nucléique dans une cellule dendritique.

Des techniques préférées dans le cadre de la présente invention sont les méthodes de sensibilisation utilisant des vésicules membranaires (de type exosome), des peptides antigéniques, des vecteurs, des ARNs ou des éluats peptidiques acides de tumeur (EPA). L'utilisation de vésicules membranaires ainsi que le "peptide pulsing" et la méthode EPA sont illustrés dans les exemples et sont tout particulièrement préférés.

### Production des dexosomes

Lorsque les populations de cellules dendritiques sont obtenues et éventuellement sensibilisées à un ou plusieurs antigènes, les dexosomes peuvent être préparés.

Cette préparation comporte une première étape, facultative, de traitement des cellules, suivie d'une seconde étape d'isolement des dexosomes.

La première étape de traitement des cellules résulte de la mise en évidence par les inventeurs que la production de dexosomes par les cellules dendritiques est un phénomène régulé. Ainsi, en l'absence de traitement, les quantités de dexosomes produites sont relativement faibles. En particulier, lorsque l'on utilise une population de cellules dendritiques matures non préalablement stimulées, la production de dexosomes est pratiquement indétectable. Les inventeurs ont donc montré que la production de dexosomes était essentiellement dépendante du type de cellules dendritiques et de la mise en oeuvre d'un traitement de ces cellules. Ce sont ces éléments préalables qui permettent d'obtenir des dexosomes ayant des propriétés avantageuses, dans des quantités significatives pour un usage industriel. Un traitement des cellules dendritiques est donc avantageusement réalisé de manière à stimuler la production de dexosomes par ces cellules. Ce traitement stimulant peut être réalisé soit par culture des cellules en présence de certaines cytokines, soit par irradiation des cellules, soit en diminuant le pH de la culture, soit en combinant ces différents types de traitement.

Dans le premier mode de mise en oeuvre, les cellules dendritiques sont incubées en présence d'une cytokine choisie de préférence parmi l'interféron gamma (IFNγ), l'interleukine-10 (IL-10) et l'interleukine-12 (IL-12), de préférence l'interféron gamma et l'IL.-10. Comme illustré dans les exemples, ces cytokines semblent exercer un effet stimulant assez prononcé sur la production de dexosomes (facteur 3 à 5). De plus, de manière surprenante, aucun effet stimulant n'a été observé en présence des cytokines suivantes : IL-1β, IL-2, IL-4, IL-6 et IL-15, et un effet inhibiteur a même été observé en présence de lipopolysaccharide (LPS) ou de TNFα, qui sont pourtant décrits comme stimulant la maturation des cellules dendritiques. Ces résultats montrent donc (i) le caractère régulé de la production de dexosomes et (ii) l'effet spécifique de certaines cytokines sur cette production. Ces résultats illustrent de plus l'intérêt surprenant d'utiliser des cellules dendritiques immatures, et l'utilisation, dans l'étape de stimulation, de cytokines induisant un état immature des cellules, telles que l'IL-10 notamment. Dans ce mode de mise en oeuvre, les cytokines sont utilisées à des doses adaptables par l'homme du métier en fonction (i) de la cytokine, (ii) de la population cellulaire et (iii) de la réalisation éventuelle d'autres traitements. Il est entendu que les cytokines sont préférentiellement utilisées à des doses sub-toxiques. Les doses d'interleukine sont généralement comprises entre 1 et 100 ng/ml, de préférence entre 1 et 50 ng/ml. L'interféron peut être mis en oeuvre à des doses comprises entre 1 et 500 Ul/ml, de préférence entre 5 et 200 Ul/ml.

Dans le second mode de mise en oeuvre, les cellules dendritiques sont soumises à une irradiation. Les résultats présentés dans les exemples montrent en effet qu'une irradiation des cellules permet également d'augmenter les niveaux de production de dexosomes. L'irradiation est généralement effectuée entre 1000 et 5000 rads, de préférence entre 2000 et 4000 rads. avantageusement autour de 3000 rads.

La seconde étape comprend l'isolement des dexosomes. Cette étape a pour but de séparer les dexosomes des cellules dendritiques et/ou du milieu de culture. Cette étape permet en particulier d'obtenir une composition enrichie en dexosomes et essentiellement dépourvue de cellules intactes. Préférentiellement, cette étape conduit à une composition comprenant 70% au moins de dexosomes, de préférence 85% au moins.

L'isolement des dexosomes peut être réalisée selon différentes techniques de séparation de matériels biologiques. Comme décrit précédemment pour les texosomes de cellules tumorales, ces techniques peuvent être basées sur les différences de taille, de masse, de charge ou de densité des dexosomes.

Ainsi, les dexosomes peuvent être isolés par centrifugation du milieu de culture ou du surnageant de culture ou de fractions membranaires ou de lysats de cellules dendritiques. Il peut s'agir par exemple d'une centrifugation différentielle et/ou d'une centrifugation en gradient de densité, suivie(s) d'une récupération de la ou des fraction(s) contenant lesdits dexosomes. Ce type de méthodologie repose sur la séparation, par centrifugations successives, des vésicules membranaires d'une part et des cellules, débris cellulaires, vésicules internes, etc., d'autre part. Dans ce mode particulier de mise en oeuvre, la fraction comprenant les dexosomes est généralement celle obtenue après ultracentrifugation à 100.000 g. Cette méthode est illustrée notamment dans les exemples 1 et 8.

L'étape d'isolement des dexosomes peut également être réalisée par chromatographie, électrophorèse et/ou nanofiltration.

Il peut s'agir avantageusement d'une électrophorèse en phase fluide et ou en gradient de densité. L'électrophorèse en phase fluide, qui permet la séparation de matériels biologiques d'après leur charge, est tout à fait avantageuse. L'exemple 11 qui suit montre en effet que cette technique peut être avantageusement utilisée pour l'isolement d'exosomes avec de bons rendements. Cette technique est par ailleurs particulièrement avantageuse sur le plan industriel.

Il peut également s'agir d'une purification par chromatographie. On peut citer notamment les chromatographies d'échange d'ions, de perméation de gel (ou d'exclusion) ou la chromatographie hydrophobe. Compte tenu de la nature lipidique des dexosomes, la chromatographie d'échanges d'ions est particulièrement intéressante. La nanofiltration peut être réalisée selon les techniques connues, à partir d'un surnageant de cellules.

Le recours à des techniques des chromatographie et/ou d'électrophorèse et/ou de nanofiltration constitue un autre aspect important de la présente invention, puisqu'il permet, par rapport aux technologies courantes, une production de qualité améliorée, dans des quantités adaptées à un usage industriel (notamment pharmacologique).

A cet égard, l'invention concerne également un procédé de préparation de vésicules membranaires comprenant au moins une étape de séparation par électrophorèse, chromatographie ou nanofiltration. Ce procédé est plus particulièrement adapté à la préparation de vésicules membranaires de type exosome, telles que les texosomes ou les dexosomes. Dans ce procédé, l'étape de séparation par électrophorèse ou chromatographie peut être réalisée directement sur un surnageant de culture, un lysat cellulaire, ou une préparation pré-purifiée. L'électrophorèse est plus préférentiellement une électrophorèse en phase fluide.

Les dexosomes présentent des propriétés remarquables qui sont illustrées dans les exemples. Ainsi, les dexosomes stimulent la prolifération de lymphocytes T cytotoxiques in vitro. De plus, in vivo, les dexosomes sont capables de bloquer la croissance tumorale. Ces vésicules sont donc capables de présenter de manière très efficace, en association avec des molécules du CMH de classe I et de classe II, des antigènes d'intérêt. Les dexosomes présentent donc. de nombreuses applications, dans le domaine du cancer, des maladies infectieuses ou parasitaires par exemple. En outre, à des doses élevées (susceptibles d'induire une tolérance), les dexosomes peuvent également être utilisées dans le traitement de pathologies telles que l'allergie, l'asthme ou les maladies autoimmunes. En outre, les dexosomes "naïfs" peuvent également être utilisés comme adjuvant pour stimuler et/ou moduler une réponse immunitaire.

L'invention a également pour objet l'utilisation:
- de dexosomes tels que définis ci-dessus,
pour la stimulation et éventuellement l'amplification *in vitro* de lymphocytes T spécifiques d'antigènes contenus dans les susdits texosomes, cellules présentatrices d'antigènes ou dexosomes, - ou de lymphocytes B, et notamment pour la stimulation et l'amplification *in vitro* de lymphocytes T.

L'invention concerne également l'utilisation de dexosomes tels que définis ci-dessus, pour la sélection ex *vivo* d'un répertoire de lymphocytes T, susceptibles de reconnaître des antigènes spécifiques contenus dans les susdits texosomes, cellules présentatrices d'antigène, ou dexosomes.

L'invention a encore pour objet un médicament comprenant à titre de substance active au moins un texosome tel que défini ci-dessus, une cellule présentatrice d'antigène telle que définie ci-dessus, et/ou un dexosome tel que défini ci-dessus, en association avec un véhicule pharmaceutiquement acceptable.

Avantageusement, l'invention concerne un médicament tel que défini ci-avant pour une utilisation dans le traitement des cancers, maladies infectieuses ou parasitaires.

Plus préférentiellement, le médicament comprend des dexosomes tels que définis ci-avant.

Selon un autre mode de mise en oeuvre, l'invention concerne un médicament tel que défini ci-avant pour une utilisation dans le traitement des pathologie de type allergie, asthme ou maladie autoimmune.

Comme forme galénique appropriée, les dexosomes peuvent être contenus dans du sérum physiologique, dans une ampoule ou toute autre moyen approprié (seringue, poche, etc). Ils peuvent être préparés extanporanément ou stockés, par exemple sous forme congelée à -80°C. Les solutions utilisées peuvent être composées de solutions salines, éventuellement supplémentées d'agents stabilisant et/ou d'adjuvants. Les agents stabilisants peuvent être notamment des protéines ou des molécules de haut poids moléculaire. On peut citer plus particulièrement des protéines telles que la sérum-albumine humaine, ou des molécules telles que le dextran ou le poloxamer par exemple.

Les compositions de l'invention peuvent également comprendre ou être utilisées en association avec un ou plusieurs adjuvants. L'adjuvant peut être plus particulièrement tout agent pharmacologique immunostimulant, tel que par exemple une cytokine (notamment interleukine-12). De tels agents sont classiquement utilisés dans les protocoles cliniques ou dans des compositions de vaccins. Par ailleurs, l'adjuvant selon l'invention peut également être un agent capable de stimuler la production de cellules dendritiques in vivo. On peut citer à titre d'exemple le composé Fit3. L'utilisation combinée de ce type d'agent permet d'augmenter le nombre de cellules dendritiques, et donc d'améliorer potentiellement l'efficacité des compositions de l'invention.

Un autre objet de l'invention concerne donc une association de dexosomes et d'un adjuvant, en vue d'une utilisation simultanée, séparée ou espacée dans le temps.

Un mode d'administration approprié des médicaments de l'invention est constitué par des injections, et notamment des injections intradermiques ou sous-cutanées. Ce mode d'administration est particulièrement adapté lorsque la substance active du médicament est constituée par des cellules dendritiques chargées en texosomes ou par des dexosomes.

Les posologies appropriées sont de 0,01 à 10, et notamment 0,10 à 5, encore plus particulièrement de 0,15 à 2 µg/kg de poids corporel, et de 10 µg pour les tests de réaction intradermiques.

Les médicaments de l'invention peuvent être également utilisés à raison de 100 µg pour les traitements de vaccinations prophylactiques.

Les objectifs visés par l'utilisation des médicaments de l'invention sont :
- l'hypersensibilité retardée (tests chez les cancéreux), ou
- la thérapie prophylactique, ou
- l'utilisation dans le cadre de la détection de la fréquence des précurseurs lymphocytaires spécifiques cytotoxiques ou sécréteurs d'interféron par la technique de dilution limite.

Il s'agit d'utiliser les cellules dendritiques autologues ou allogéniques préincubées avec les texosomes de l'invention, comme cibles de lymphocytes périphériques de sujets porteurs de la tumeur, avant, pendant et après traitement anti-tumoral (traitement classique ou immunisation active spécifique).

L'invention concerne également l'utilisation d'un dexosomes tel que défini ci-dessus, pour la préparation d'un médicament destiné au traitement de tumeurs, notamment solides, ascitiques et hématopoïétiques.

Comme tumeurs solides, on peut citer : le cancer du rein, du sein, du colon, du poumon, de l'estomac, du foie, les mélanomes, sarcomes, etc...

Comme tumeurs hématopoïétiques, on peut citer : les leucémies, les lymphomes malins hodgkiniens ou non hodgkiniens.

Comme indiqué précédemment, les compositions de l'invention, notamment les compositions comprenant des dexosomes, sont également utilisables pour le traitement des maladies infectieuses ou parasitaires. Pour ce type d'application, les dexosomes sont chargés en antigènes ou peptides de l'agent infectieux (virus) ou du parasite.

L'invention concerne également l'utilisation d'un dexosome tel que défini ci-dessus, dans le cadre d'un test d'hypersensibilité retardée du cancer ou encore comme outil diagnostique de recherche de fréquence de précurseurs spécifiques CTL cytotoxiques.

L'invention concerne également l'utilisation d'un dexosome tel que défini ci-dessus, pour le transfert de matériel biologique dans une cellule *in vitro* ou *in vivo.*

La création de banques de texosomes dérivés de cellules tumorales de type histologique commun ou différents est envisagée.

Celles-ci sont composées de mélanges de texosomes faits à partir de lignées de cellules tumorales pour un type de cancer donné. Ces banques de texosomes peuvent permettre de sensibiliser des cellules présentatrices d'antigène, notamment des cellules dendritiques, contre toute les tumeurs de ce type.

On peut citer des mélanges de texosomes pour des tumeurs génétiquement reliées (cancer du sein et de l'ovaire) ou présentant des mutations p53, p16 connues (cancer du sein, sarcome).

On peut également mentionner des mélanges de texosomes tumoraux avec des vésicules provenant de cellules immortalisées et transfectées pour exprimer des molécules de co-stimulation, des molécules d'adhésion, des chemokines attractantes (différentes de celles exprimées sur les texosomes).

La présente invention sera décrite plus en détails à l'aide des exemples qui suivent, qui doivent être considérés comme illustratifs et non limitatifs.

### LÉGENDES DES FIGURES

**Tableau 1.** Les cellules de lignées tumorales ont été incubées pendant 24 h à une densité de un million de cellules par millilitre. Les texosomes ont ensuite été préparés (voir exemple) à partir des milieux de culture par ultracentrifugation différentielle. La concentration en protéines texosomales est mesurée par le test de Bradford (BioRad Protein Assay [BioRad].

MZ-2 est décrite dans Traversari et al., (1992)

Les * signifient que les différentes lignées primaires ont été établies et caractérisées dans le laboratoire de biologie clinique de l'Institut Gustave Roussy et sont accessibles sur demande.

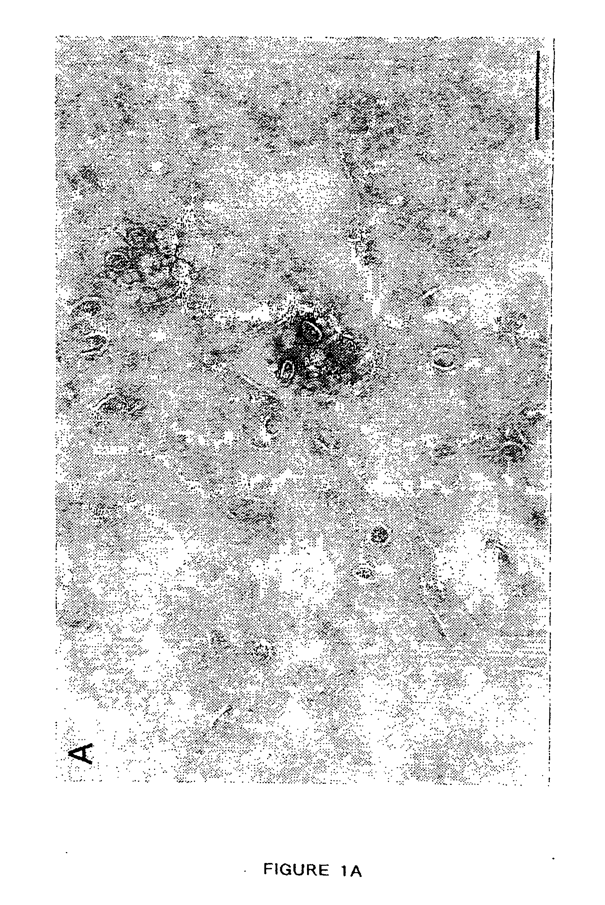

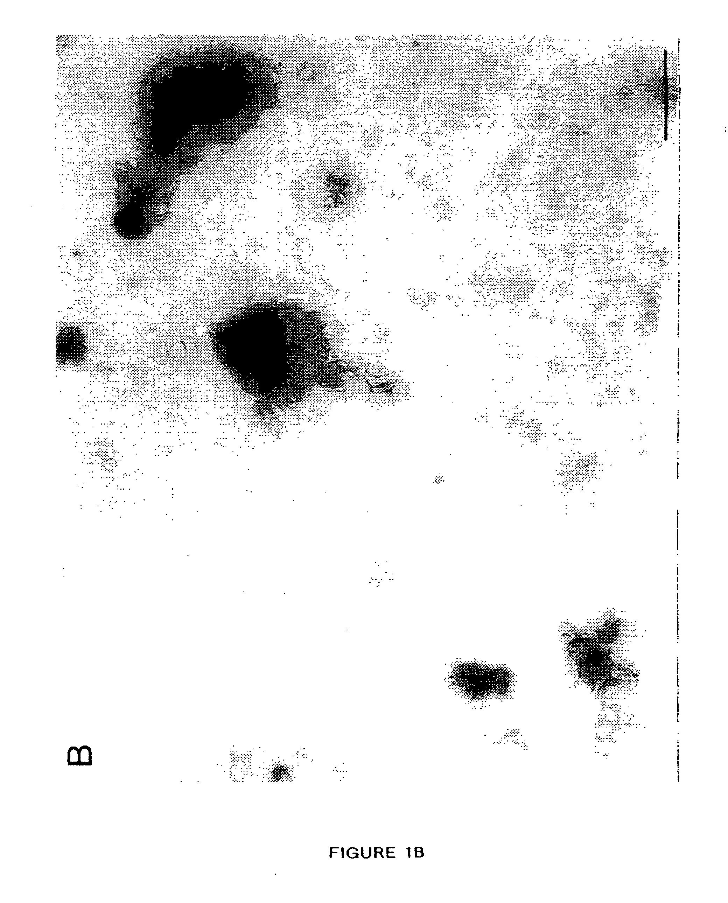

**Figures 1A et 1B.** Morphologie des endosomes multi-vésiculaires et des texosomes dérivés de cellules TS/A.
A. Sections ultrafines des cellules TS/A analysées par microscopie électronique. Détail du cytoplasme montrant un compartiment endosomal contenant des vésicules de 60-80 nm de diamètre.
B. Préparation de texosomes de cellules TS/A analysées en microscopie électronique par la technique sur vésicule intacte (Raposo et al. (1996)). Les préparations de texosomes contiennent une population majoritaire de vésicules de 60-80 nm de diamètre, de taille et morphologie similaires aux vésicules internes des endosomes multivésiculaires montrés en A.

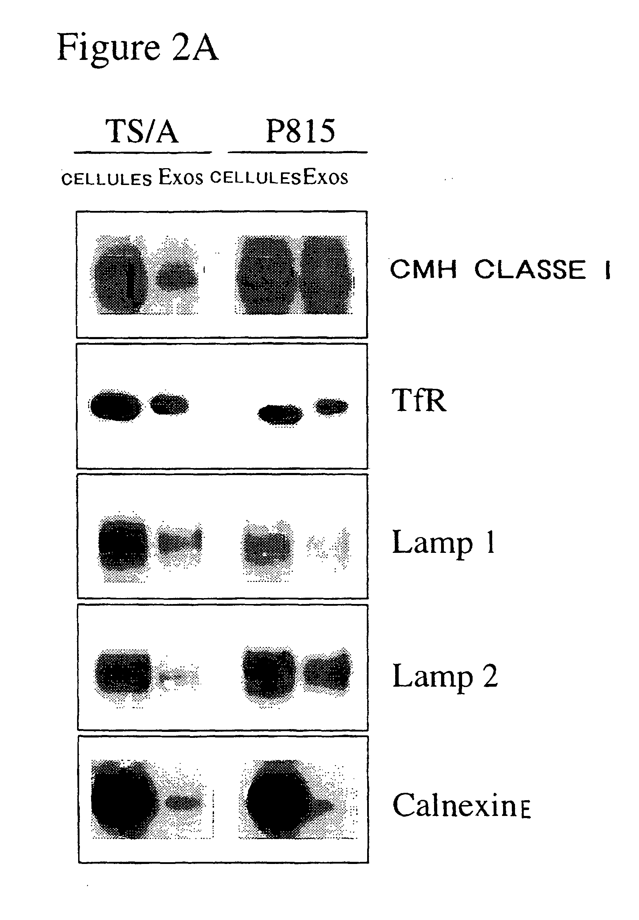

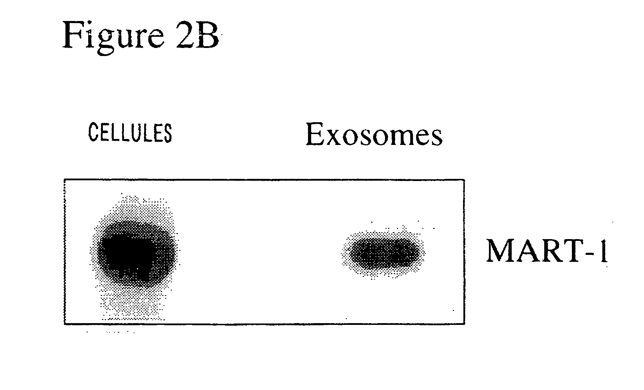

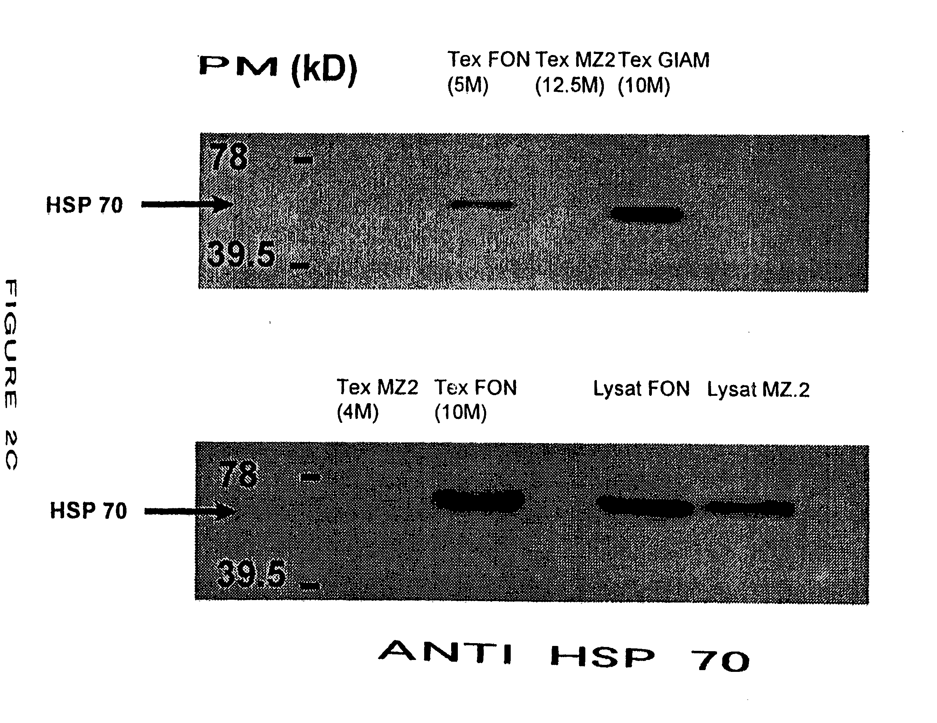

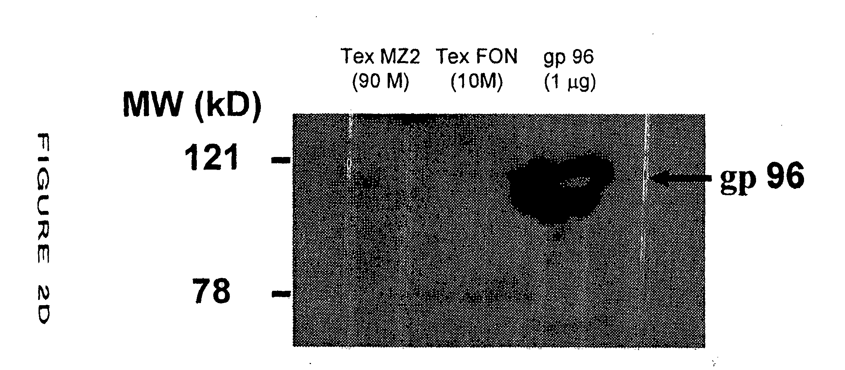

**Figure 2.** Présence de différents marqueurs dans les texosomes de cellules tumorale.
A. Deux microgrammes de protéines texosomales (Exos) ou 2x10⁵ cellules tumorales ont été analysés par Western Blot à l'aide d'anticorps monoclonaux spécifiques de : molécules de classe I du CMH (Machold Robert P. et al. (1995) "Peptide Influences the Folding and Intracellular Transport of Free Major Histocompatibility Complex Class I Heavy Chains" J. Exp. Med. 181 : 1111-1122), récepteur de la transférine (TfR) (anticorps correspondant étant H68.4 décrit dans Biochimica et Biophysica Acta (1992) 1136(1): 28-34), Lamp 1 et 2 (rat anti-mouse monoclonal antibodies, Pharmingen) et Calnexine (Hebert Daniel N. et al. (1995) "Glucose Trimming and Reglucosylation determine Glycoproteine Association with Calnexin in the Endoplasmic Reticulum" Cell 81: 425-433).
B. Dix microgrammes de protéines texosomales d'une lignée de cellules de mélanome (FON) ou 10 µg de protéines totales des mêmes cellules ont été analysés par Western Blot à l'aide d'un anticorps anti-MART-1 (Marincola F. et al. (1996) "Analysis of expression of the melanoma associated antigens MART-1 and gp100 in metastatic melanoma cell lines and in "in situ" lesions" Journal of Immunotherapy 19:192-205).
C. Les protéines texosomales d'une lignée de cellules de mélanome (FON) ou les protéines totales des mêmes cellules ont été analysées par Western Blot à l'aide d'un anticorps anti-HSP70.
D. Les protéines texosomales d'une lignée de cellules de mélanome (FON) ou de la lignée MZ-2 ont été analysées par Western Blot à l'aide d'un anticorps anti-gp96.

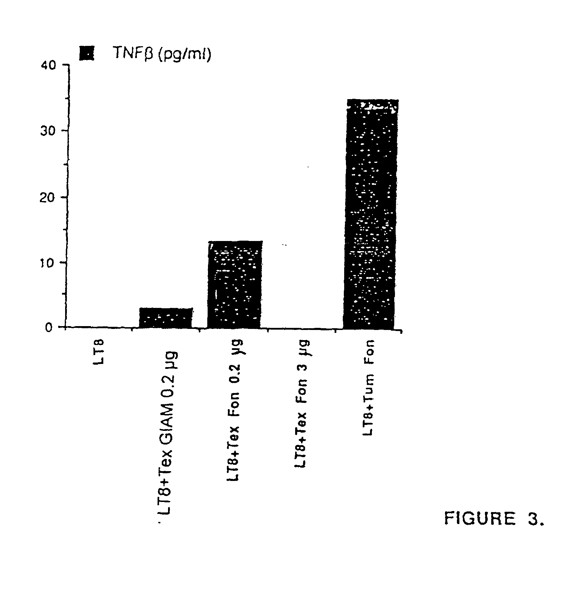

**Figure 3.** Les texosomes dérivés d'une lignée tumorale MART-1 positive (FON) stimulent un clone T spécifique de MART-1.

Vingt mille cellules du clone T LT8 (ou LT12, résultats non montrés) ont été incubées avec des texosomes dérivés de FON (lignées de cellules MART-1 et HLA-A2 positive), ou de GIAM (cellules d'une lignée de néphrome, MART-1 négative) comme contrôle négatif, pendant 48 h. La production de TNFβ par les cellules du clone T a été mesurée par essai biologique avec les cellules WEHI (Espavik et al.). Les texosomes induisent la production d'IFNγ par le clone T, révélant ainsi la présence de complexes HLA-A2/peptide dérivés de MART-1 à la surface des texosomes.
- "LT8+TexGIAM" correspond à des clones T LT8 incubés en présence de texosomes dérivés de cellules tumorales GIAM ;
- "LT8+TexFON" correspond à des clones T LT8 incubés en présence de texosomes dérivés de cellules tumorales FON ;
- "LT8+TumFON" correspond à des clones T LT8 incubés en présence de texosomes dérivés de cellules tumorales FON ;

En abscisses, on a représenté les cellules conditionnées et en ordonnées la quantité produite de TNFβ (pg/ml).

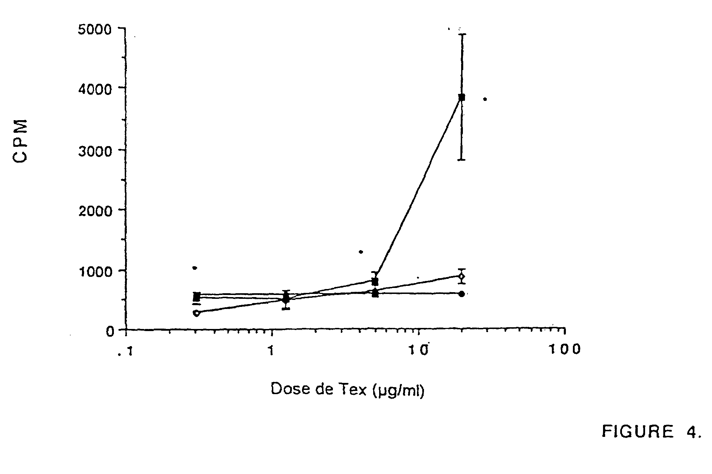

**Figure 4.** Les texosomes de cellules P815 exprimant la βGal stimulent des splénocytes de souris immunisées par des adénovirus βGal recombinants.

Des splénocytes (10⁵) de souris BALB/c immunisées 2 mois avant avec 10⁶ pfu adénovirus recombinant βGal ayant rejeté une tumeur exprimant βGal ont été incubés avec des texosomes dérivés des cellules P815 (carreaux vides ◇) ou des cellules P815-βGal (carrés pleins **■).** Les splénocytes non incubés en texosomes donnent le bruit de fond symbolisé par des points pleins (●). Après 5 jours de culture, 1 µCi de thymidine tritiée a été ajouté par puits de culture. L'incorporation de tritium dans l'ADN cellulaire a été mesurée 18 h plus tard. Les résultats significativement différents d'après la méthode exacte de Fisher sont marqués *.

En abscisses, on a représenté la quantité en texosomes dérivés des cellules tumorales P815 (µg/ml) et en ordonnées les coups par minute (CPM).

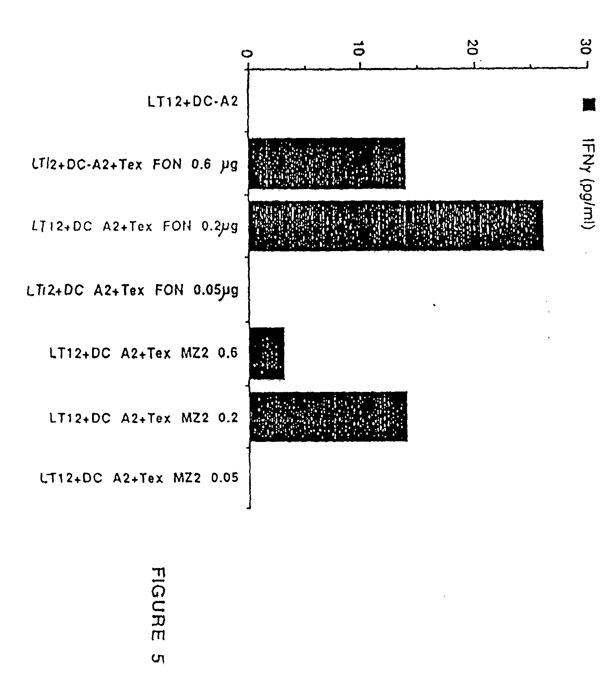

**Figure 5**. L'antigène tumoral MART-1 contenu dans des texosomes peut être présenté aux lymphocytes T par des cellules dendritiques.

Des texosomes à doses croissante dérivés des lignées de cellules tumorales FON (MART-1+-, HLA-A2+, A1-) et MZ2 (MART-1+, HLA-A2-, A1+) ont été incubés avec les clones (20 000 cellules par puits de 96 microplaques) T LT12 (spécifique de HLA-A2/peptide de MART-1) en présence de cellules dendritiques HLA-A2+ dérivées de macrophages circulants (DCA2) (Sallusto, F. And Lanzavecchia A., 1994, Efficient présentation of soluble antigen by cultured human dendritic cells is maintained by GMCSF et IL-4 and down regulated by TNFα. J. Exp.Med. 179:1109-1118). La sécrétion d'IFN_{γ} représentée en ordonnées (pg/ml) a été mesurée dans les surnageants de culture après 2 jours. Les texosomes dérivés de FON, ainsi que ceux dérivés de MZ2, ont induit la sécrétion d'IFN_{γ} par LT12 et LT8 (résultats non montrés). Les cellules FON ont aussi induit une forte sécrétion d'IFNγ par les clones T, alors que les cellules MZ2, qui n'expriment pas l'haplotype de molécule HLA adéquate (HLA-A2), n'ont pas induit de production d'IFNγ.
- "LT12+DCA2" correspond à des clones T LT12 incubés en présence de cellules dendritiques HLA-A2+ ;
- "LT12+DCA2+TexFON" correspond à des clones T LT12 incubés en présence de cellules dendritiques chargées en texosomes dérivés de cellules tumorales FON ;
- "LT12+DCA2+TexMZ2" : correspond à des clones TL12 incubés en présence de cellules dendritiques chargées en texosomes dérivés de cellules tumorales MZ2.

**Figures 6A et 6B.** Effets anti-tumoraux des texosomes in *vivo.*

Cent mille cellules tumorales TS/A ont été injectées par souris BALB/c (A) ou Nude (B). Trois jours plus tard, chaque souris a reçu deux injections successives, à 24 h d'intervalle (représentées par J3 et J4 sur la figure), de 20 µg à 30 µg de texosomes en intradermique. La taille des tumeurs a ensuite été mesurée deux fois par semaine. Les analyses statistiques ont été faites par la méthode exacte de Fisher (la significativité à 95 % est indiquée par une*).
A. Deux groupes de 5 souris ont reçu des texosomes dérivés de TS/A (triangles pleins) ou de MCA38 (triangles vides Δ) (une lignée de cellules d'adénocarcinome de colon dérivé d'une souris C57BL/6), comme contrôle négatif. Seuls les texosomes dérivés de TS/A ont un effet anti-tumoral *in vivo.*
B. Deux groupes de souris Nude ont reçu en parallèle les mêmes doses des mêmes préparations de texosomes (■ : exosomes de TS/A; □ : exosomes de MC38).

Aucun effet anti-tumoral n'a été observé sur les souris Nude. Les lymphocytes T sont donc nécessaires aux effets anti-tumoraux des texosomes *in vivo.*

En abscisses, on a indiqué les jours et en ordonnées la taille moyenne de la tumeur (mm²).

**Figure 7.** Des cellules dendritiques dérivées de moelle osseuse sensibilisées par les texosomes dérivés de cellules tumorales induisent l'éradication totale in vivo de tumeurs solides établies.

Cinq cent mille cellules tumorales P815 ont été injectées dans le flanc droit des souris DBA/2 10 jours avant le traitement. Le traitement a consisté en une seule injection de texosomes (10 µg/souris) dans le même flanc, mais à distance de la tumeur. Un autre groupe a été injecté en intraveineux par des cellules dendritiques (dérivée de moelle osseuse par traitement pendant 5 jours en GM-CSF+IL4 (Mayordomo et al., 1995), incubées au préalable pendant 3 h avec les texosomes de P815. Les tumeurs ont été mesurées et les résultats analysés comme décrit dans la figure 6. Les texosomes de P815 ont sensibilisé les cellules dendritiques pour induire le rejet des tumeurs établies. Les microsomes n'ont pas eu d'effet significatif sur la croissance tumorale. L'insert montre le pourcentage de souris sans tumeur (en ordonnées), la barre pleine correspond uniquement aux groupes d'animaux de type carrés pleins, l'abscisse correspond aux jours. Ces souris n'ont pas développé de tumeur après ré-injection du double de la dose tumorale minimum, montrant qu'elles avaient développé une immunité anti-tumorale. Les symboles utilisés dans la figure sont les suivants :
○ : cellules dendritiques incubées avec des texosomes témoins,
■ : cellules dendritiques incubées avec des texosomes P815, triangles pleins : texosomes P815 en intradermique,
X : animaux non traités.

En abscisses, on a indiqué les jours et en ordonnées le volume moyen de la tumeur.

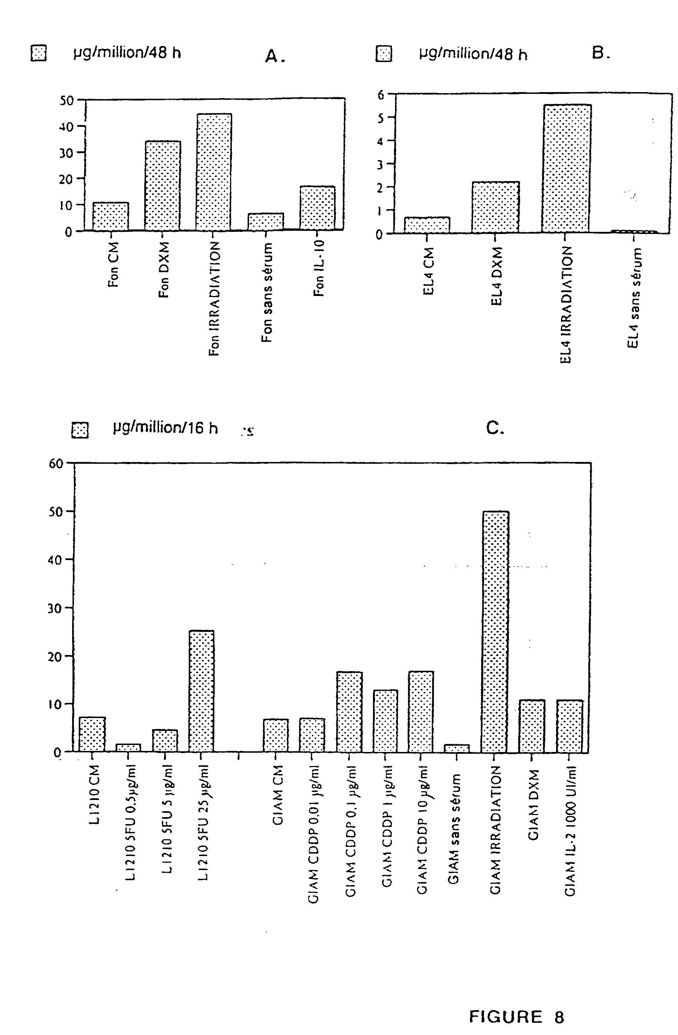

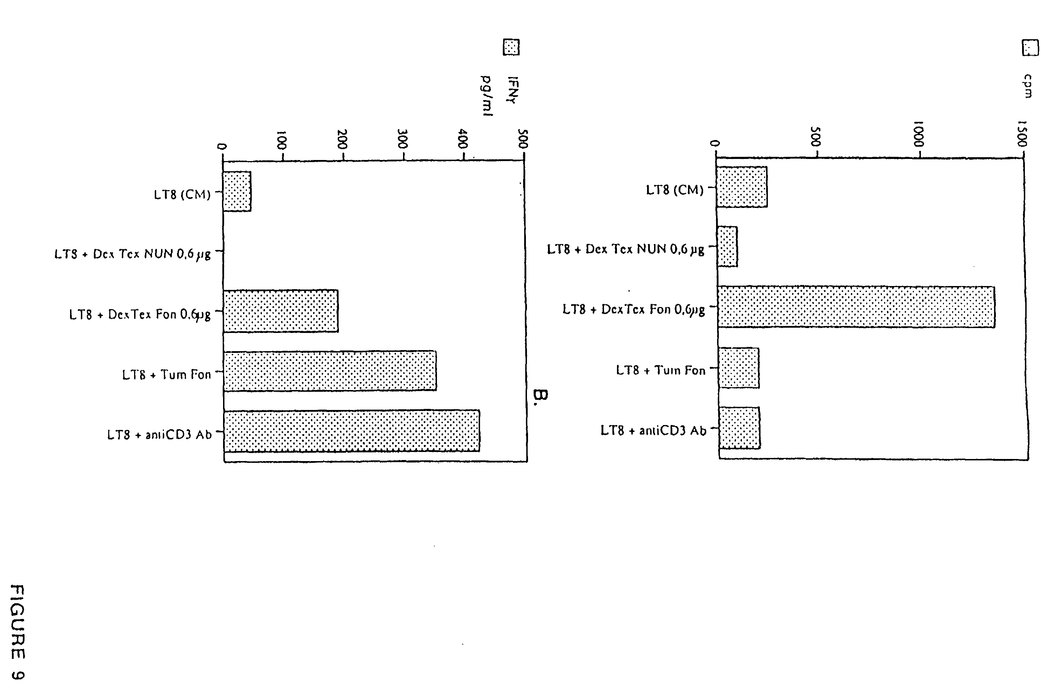

**Figures 8A, 8B et 8C.** Des agents chimiothérapeutiques/cytotoxiques et l'irridiation peuvent stimuler l'exocytose des texosomes tumoraux.

La leucémie murine L1210 et la lignée de cancer du rein primitif humain GIAM ont été utilisées (figure 8C). Deux millions de cellules tumorales ont été incubées en présence de quantités croissantes de 5 Fu (pour L1210) ou de cis-platinum (CDDP) (pour GIAM) par ml pendant 16 heures.

Le surnageant a été récupéré, puis a fait l'objet d'ultracentrifugations différentielles comme décrit dans le Tableau 1.

GIAM a aussi été incubée en IL-2 1000 Ui/ml, ou en dexamethasone (10⁻⁶ M), ou irradiée à 10.000 rads.

Les fortes doses de chimiothérapie et l'irridiation sont de bons exemples de régulation positive d'exocytose des texosomes dans ces modèles tumoraux. Les résultats ont été retrouvés dans d'autres tumeurs également (figures 8A et 8B) ; en particulier, l'irridiation semble être le stimulus d'exocytose le plus puissant.

La figure 8A correspond au mélanome FON sus-décrit et la Figure 8B correspond à un lymphome murin désigné par EL4 (J. Nat. Cancer Ins. (1972) 48 : 265-271).

Sur la. figure 8A, on a représenté les cellules FON incubées dans différentes conditions :
1) CM : milieu de culture de base (RPMI contenant 10% de sérum de veau foetal),
2) DXM = en présence de dexaméthasone,
3) irradiation : irradié à 10.000 rads,
4) sans sérum,
5) IL-10 = en présence d'IL-10, à raison de 10 ng/ml.

Les irradiations ci-dessus sont valables également pour les cellules EL4, L1210 et GIAM.

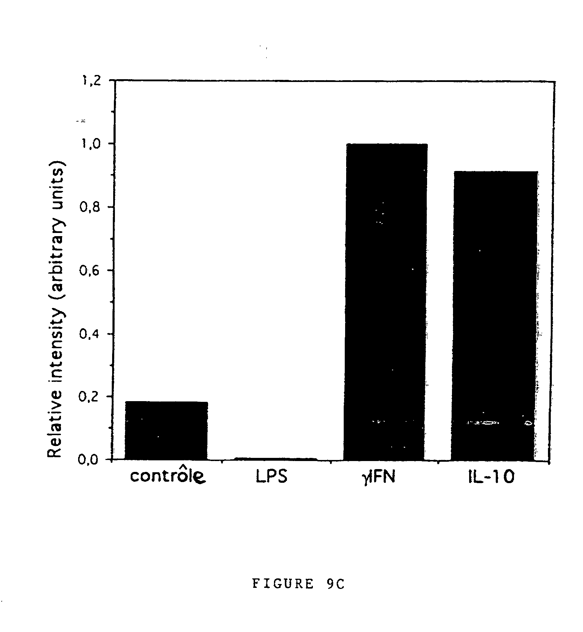

**Figures 9A, 9B et 9C.** Les vésicules membranaires (dexosomes) produites par les cellules dendritiques sensibilisées à des antigènes tumoraux de mélanomes sont efficaces pour stimuler les lymphocytes T spécifiques de ces mélanomes, et leur production est régulée par les cytokines.
- LT8 (CM) correspond aux clones T LT8 incubés en milieu de culture de base comme défini à la figure 8A,
- DexTexNUN correspond au dexosome dérivé de cellules dendritiques chargées en texosome provenant de cellules tumorales NUN,
- DexTexFON correspond au dexosome dérivé de cellules dendritiques chargées en texosome provenant de cellules tumorales FON,
- TumFON correspond aux cellules tumorales FON.

A. Essais de prolifération : les texosomes Fon (utilisés figure 3) ont été incubés avec des cellules dendritiques HLA-A2 pendant 3 heures, puis lavés en solution saline à 9%. puis incubés en milieu acide pH6.3 pendant 18 heures. Les vésicules membranaires de cellules dendritiques (dexosomes) sont ainsi récupérées du surnageant de culture des susdites cellules dendritiques HLA-2.
   Les vésicules membranaires provenant de cellules dendritiques chargées avec des texosomes (DexTex) sont alors incubées avec les clones LT8 spécifiques de MART-1 présentés dans le contexte HLA-A2 (les texosomes Fon contiennent l'antigène MART-1). Comme contrôle négatif, les texosomes Nun (lignée de cancer du rein HLA-A2 négative, MART-1 négative) ont été utilisés. Comme contrôle positif, on a utilisé la lignée tumorale irradiée FON (TumFON) ou l'anticorps anti-CD3, préasorbé sur plastique (anti-CD3Ab). L'incubation des DexTex avec les clones LT8 dure 48 heures, puis 1 µCi de thymidine tritiée est ajouté par puits de 200 µl. Les proliférations lymphocytaires LT8 sont mesurées 18 heures plus tard.
   En ordonnées, on a représenté les coups par minute.
B. Mêmes manipulations, mais l'IFNγ est mesuré dans le surnageant de culture à 48 heures, par ELISA. En ordonnées, on a représenté la teneur en IFN_{γ} (pg/ml).
C. Les dexosomes ont été isolés à partir de sumageants de cellules dendritiques après 48 heures d'incubation en présence ou en absence de LPS (20 µg/ml), IFN-γ (100 Ul/ml) ou IL-10 (10µg/ml).

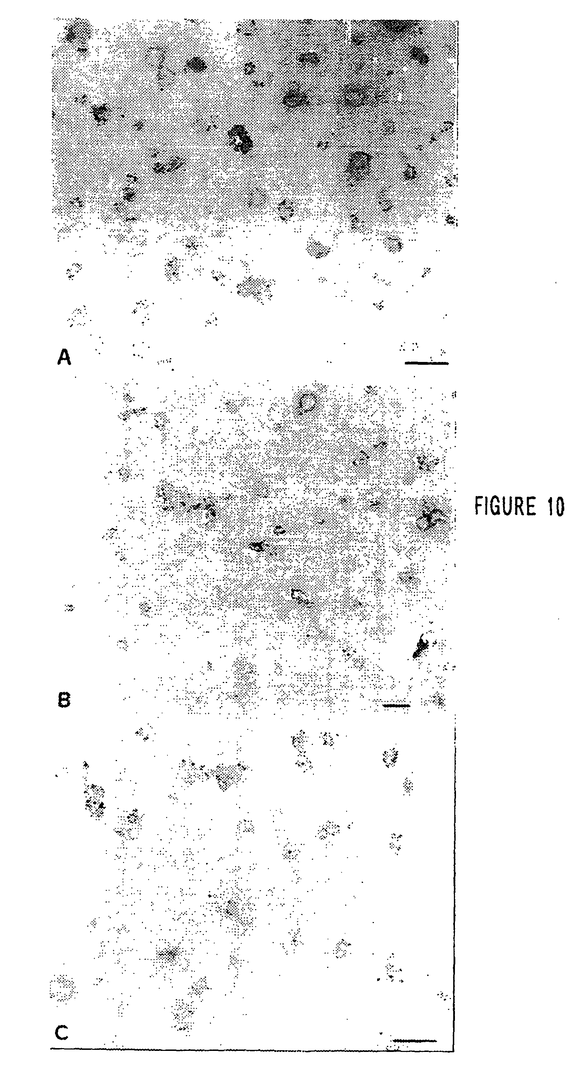

**Figure 10.** Images en microscopie immuno-electronique de dexosomes. Les dexosomes possèdent un diamètre homogène compris entre 50 et 90 nm et sont marqués de manière intense par des anticorps anti-CD63 (Figure 10A). La majeure partie de ces dexosomes est également marquée par des anticorps anti-MHC-I (Page 15, Figure 10B) et anti-MCH-II (Page 15, Figure 10C). Barres : 250 nm.

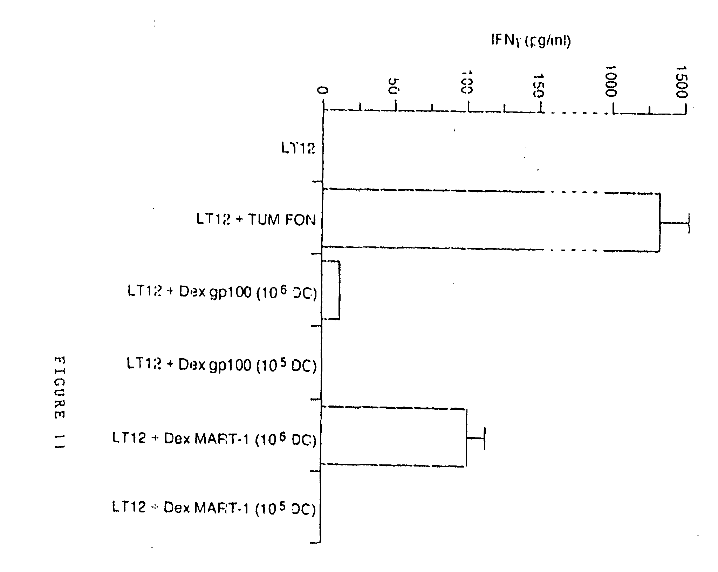

**Figure 11**. Mesure des niveaux d'interféron gamma sécrétés par les lymphocytes T incubés en présence de dexosomes chargés en peptides ou de dexosomes contrôles. Les cellules dendritiques (2X10⁶ /ml) ont été incubées pendant 3 à 12 heures, soit en présence de 10 µg/m) du peptide antigénique MART-1/MelanA₍₂₇₋₃₅₎, soit en présence de 10 µg/ml de peptide gp100₍₂₈₀₋₂₈₈₎ (contrôle) en suspension dans l'acide citrique à pH 3,7, puis les dexosomes ont été isolés. Les cellules du clone LT12 (clone de CTL HLA-A2 restreint, MART-1(27-35) spécifique) ont alors été incubées (100 000 CTL par puits) avec des doses croissantes de dexosomes ou de peptides gp100 (contrôle) dans des plaques 96 puits pendant 5 jours. La sécrétion d'interféron gamma par les cellules a ensuite été mesurée par ELISA (Genzyme).

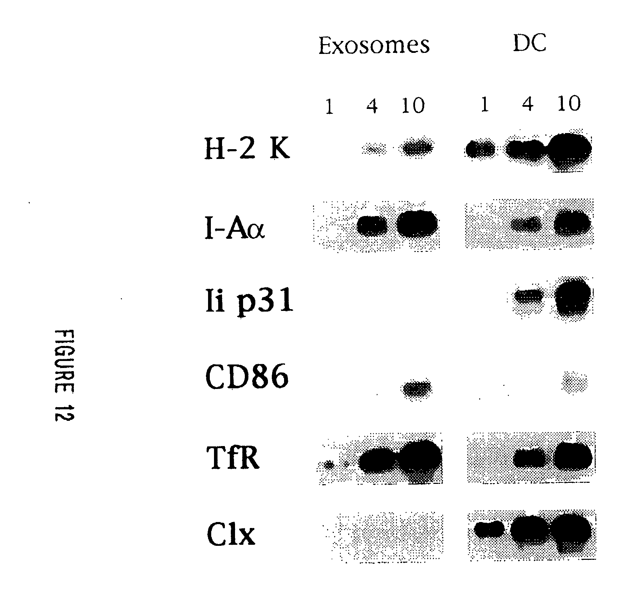

**Figure 12**. Analyse en Western Blot des marqueurs présents sur les dexosomes (1,4 et 10 µg) produits par des cellules dendritiques dérivées de moelle osseuse : H-2K (MHC-I), l-Aα (MHC-II), CD86, CD63, TfR (récepteur transferrine), Clx (Calnexine), li p31 (chaîne invariable).

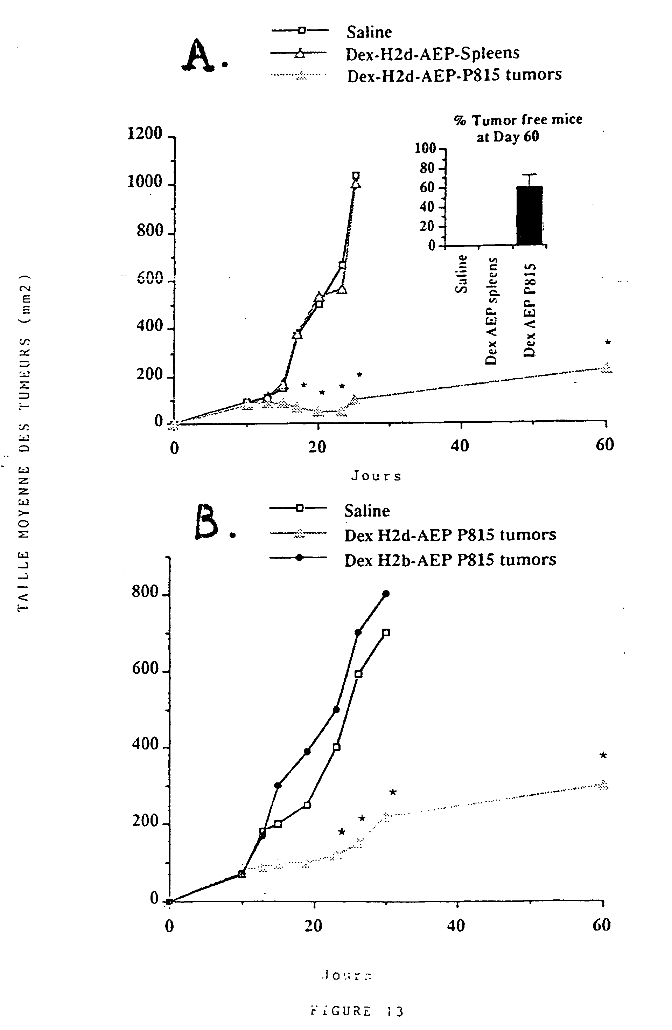

**Figure 13.** Effet anti-tumoral in vivo des dexosomes sur un modèle de tumeur du mastocytome (P815). Dex-H2d-AEP-P815 : Dexosomes dérivés de cellules dendritiques de moelle osseuse chargées en eluat peptidique acide de la tumeur P815. Dex-H2d-AEP-Spleens : Dexosomes dérivés de cellules dendritiques de moelle osseuse chargées en eluat peptidique acide de la rate.

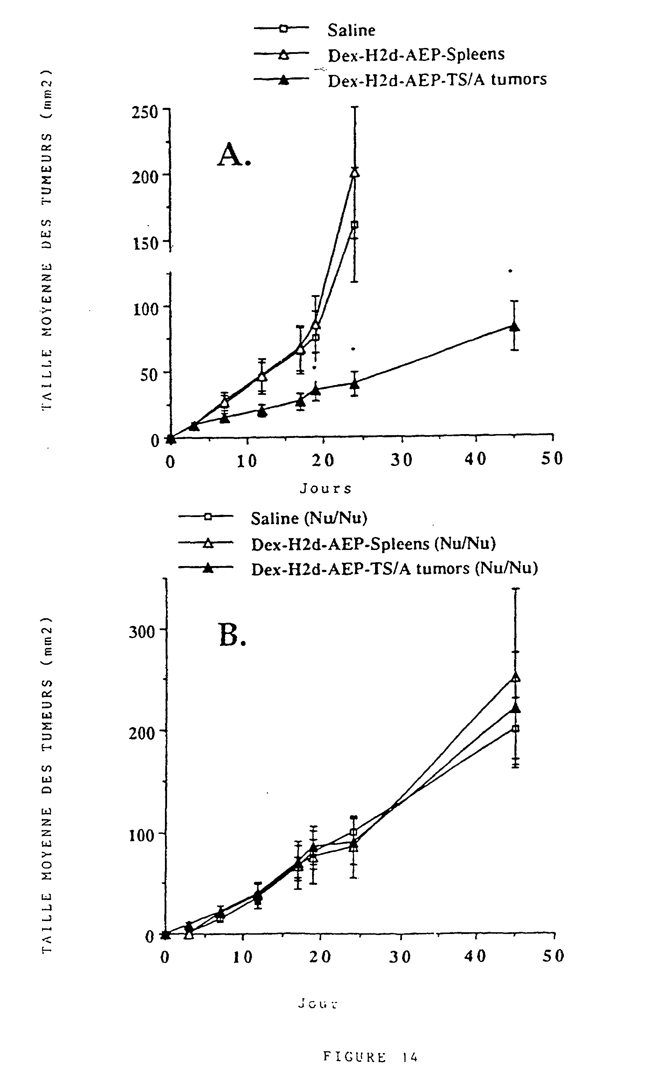

**Figure 14.** Effet anti-tumoral in vivo des dexosomes dérivés de cellules dendritiques de moelle osseuse chargées en eluat peptidique acide de tumeur sur un modèle de tumeur mammaire (TS/A). Légende : voir Figure 13. (A) Expérience réalisée sur des souris immunocompétentes. (B) Expérience réalisée sur des souris Nude.

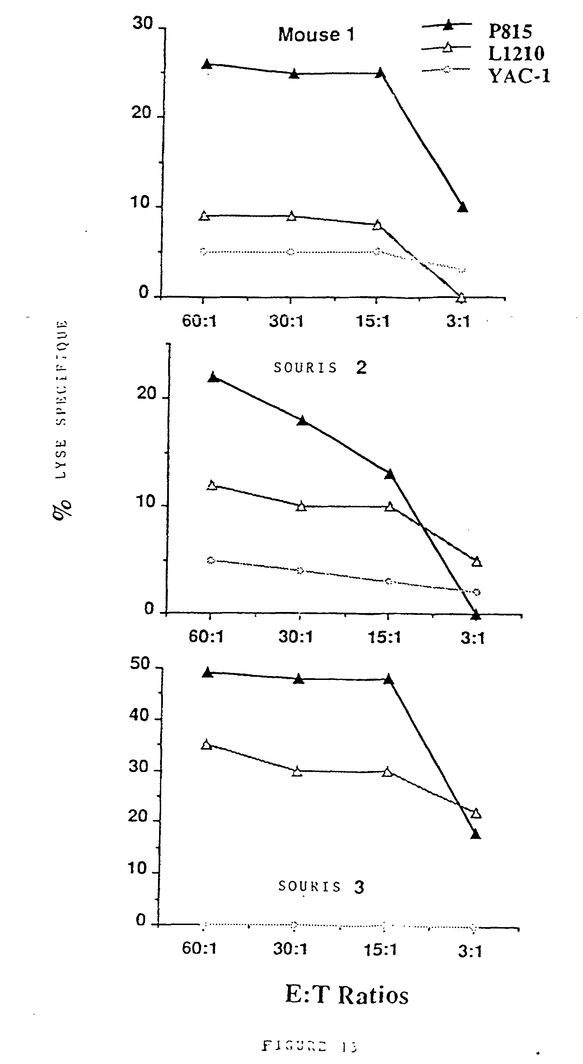

**Figure 15.** Test de libération du chrome radioactif (51Cr). Ce test permet de montrer que les dexosomes de l'invention déclenchent une réponse CTL spécifique in vivo. Cellules cibles : P815, lignée leucémique L1210. lignée YAC insensible aux cellules NK.

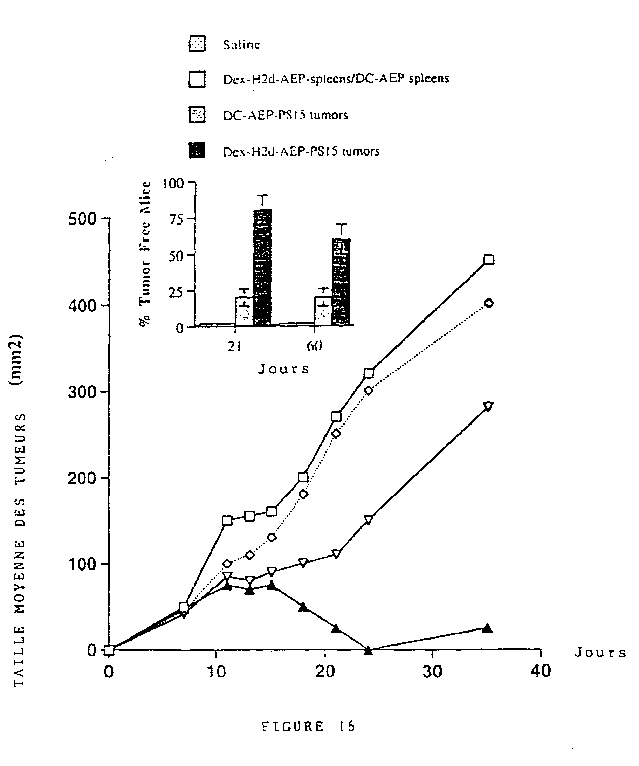

**Figure 16.** Efficacité comparée des dexosomes et des cellules dendritiques. Cette figure montre que les dexosomes sont plus puissants que les cellules dendritiques immatures dont ils dérivent pour éradiquer des tumeurs établies in vivo. 5 millions de cellules dendritiques chargées en eluat peptidique acide de la rate (carreaux vides) ou de la tumeur P815 (triangles vides) ont été administrées par voie intraveineuse ou intradermique à des souris présentant des tumeurs établies P815 au jour 8 à 10. En parallèle, le surnageant de ces cellules a été récolté après incubation 18h avec les peptides de la rate (carreaux vides) ou de la tumeur P815 (triangles pleins), ultracentrifugé et caractérisé pour son contenu en dexosomes. 5 millions de cellules dendritiques ont permis d'obtenir de 5 à 10 µg de dexosomes, qui ont permis l'immunisation de 5 souris par administration intradermique dans le flanc ipsilatéral. Une administration unique de dexosome a été réalisée au jour 8-10. La taille des tumeurs a été mesurée deux fois par semaine et est représentée sur la figure, les (*) représentent des résultats significatifs à 95% selon la méthode exacte de Fisher, en comparaison avec les injections de solution saline (carrés vides) ou de cellules dendritiques pulsées. Dans l'encart sont représentés les pourcentages de souris immunisées contre P815 montrant une absence totale (disparition) de tumeur pendant (jour 21) et à la fin (jour 60) de l'expérience, dans les différents groupes de 5 souris.

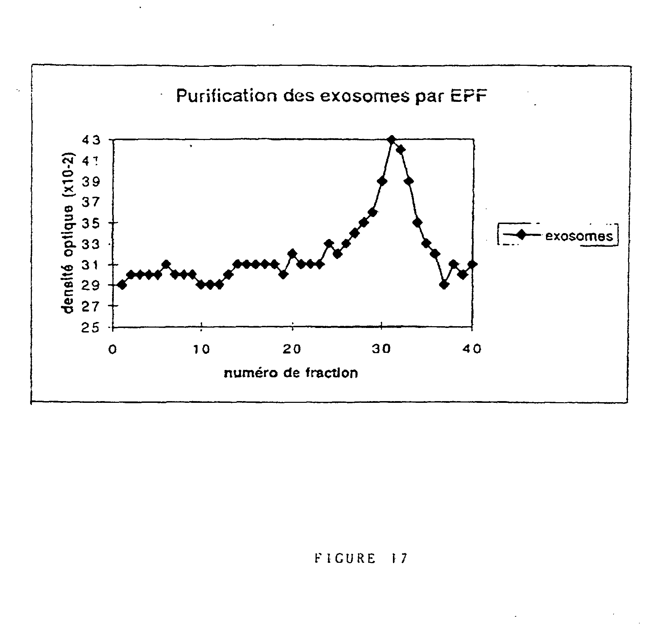

**Figure 17.** Purification des dexosomes par électrophorèse en phase fluide.

### EXEMPLES

### 1. Production de texosomes par des lignées de cellules tumorales humaines et murines.

Cet exemple illustre la capacité des cellules tumorales à produire des vésicules lipidiques.

Les cellules tumorales murines ou humaines provenant de leucémies ou de tumeurs solides (rein ou mélanome du colon) (voir tableau 1) ont été incubées pendant 24 h à une densité de un million de cellules par millilitre. Le milieu de culture (RPMI contenant 10% de sérum de veau foetal) a ensuite été débarrassé des cellules par centrifugation à 300 g pendant 10 minutes. Les débris cellulaires ont ensuite été éliminés par deux centrifugations successives de 15 min. chacune à 800 g (et une centrifugation éventuelle de 30 minutes à 10 000 g). Les texosomes ont enfin été recueillis par centrifugation de 60 minutes à 100 000 g, puis lavés une fois en PBS dans les mêmes conditions. La concentration en protéines dans les préparations de texosomes a été mesurée par la méthode de Bradford (BioRad Protein Assay [BioRad]).

Toutes les lignées tumorales testées, humaines et murines (solides ou hématopoïétiques, primaires ou établies en culture ou provenant de tumeurs fraîches dissociées), produisent des texosomes (Tableau 1). Cependant, les efficacités de production sont variables entre différentes lignées. Les lignées de cellules tumorales murines produisent entre 100 et 200 microgrammes de protéines de texosomes par 50 millions de cellules en 24 heures. Les lignées humaines de mélanome et de néphrome produisent entre 10 et 100 microgrammes de protéines de texosomes par 20 millions de cellules en 24 heures.

### 2. Les vésicules produites par les cellules tumorales sont d'origine endocytique.

Afin de déterminer si les vésicules purifiées à partir des sumageants des lignées de cellules tumorales sont d'origine endocytique, nous avons réalisé une étude morphologique par microscopie électronique de l'une de ces lignées tumorales, TS/A (lignée de carcinome mammaire de souris) (Nanni P. et al. (1983) "TS/A: a new metastasizing cell line originate from a BALB/c sponteneous mammary adenocarcinoma" Clin. Exp. Metastasis 1:373-380). Les cellules tumorales ont été fixées et apprêtées pour la microscopie électronique comme décrit précédemment. Les texosomes ont été directement déposés sur les grilles et analysés.

La figure 1A montre des exemples de compartiments intracellulaires d'aspect multivésiculaire observés dans les cellules tumorales. Ces compartiments endocytiques ont un diamètre de 200-300 nm (la barre au bas des panneaux A et B représente 200 nm) et sont composés d'une membrane externe entourant de multiples vésicules internes de 68-80 nm de diamètre. Les préparations de texosomes contiennent une population majoritaire de vésicules de 60-80 nm de diamètre (figure 1B), quelques fois agrégées, et de morphologie semblable aux vésicules internes des endosomes multivésiculaires observés à l'intérieur des cellules (Figure 1A). Ces résultats suggèrent que les texosomes sont sécrétés dans le milieu extracellulaire après fusion de la membrane externe des endosomes avec la membrane cytoplasmique. En effet, de tels profils d'exocytose sont observés dans ces cellules (données non montrées).

Afin de déterminer si les texosomes sont effectivement d'origine endocytique, on a ensuite effectué une analyse par Western Blot des marqueurs présents définis ci-après dans les texosomes dérivés de lignées tumorales, TS/A et P815 (mastocytome données par T. Boon, Ludwig institute, Bruxelles,. Belgique) (mastocytome murin). Pour ce faire, deux microgrammes de protéines de texosomes ou le lysat cellulaire de 200 000 cellules TS/A ont été séparés par gel de polyacrylamide, puis transférés sur une membrane de Nylon (Amersham). La présence éventuelle de différents marqueurs a ensuite été révélée à l'aide d'anticorps spécifiques. Les texosomes de TS/A et de P815 contiennent des molécules de classe I du CMH, ainsi que différents marqueurs de la voie endocytique (le récepteur de la transférine, les glycoprotéines lysosomales Lamp 1 et 2) (figure 2A). Par contre, un marqueur caractéristique du Reticulum Endoplasmique (RE), la calnexine, n'est pas présent dans les préparations de texosomes, montrant que des membranes du RE ne contaminent pas les texosomes.

### 3. Les texosomes produits par une lignée de mélanome contiennent un antigène turmoral cytosolique.

Ces résultats montrent que les texosomes sécrétés par les cellules tumorales correspondent aux membranes internes des endosomes multivésiculaires. Or, ces vésicules intraendosomales se forment par invagination, puis bourgeonnement de la membrane externe des endosomes vers l'intérieur de l'endosome. Ces vésicules intraendosomales et par voie de conséquence les texosomes, devraient contenir une fraction de cytosol. Ceci est particulièrement important dans le cadre de l'immunothérapie anti-tumorale, puisqu'un certain nombre d'antigènes tumoraux, y compris MART-1 (l'un des plus étudiés), sont des protéines cytosoliques. On a donc testé la présence de MART-1 dans les texosomes.

Pour ce faire, dix microgrammes de protéines de texosomes ou le lysat cellulaire de 200 000 cellules d'une lignée de mélanome humain (M10) (T. Boon, Ludwig Institute, Bruxelles, Belgique) ont été analysés par Western Blot, comme précédemment. La présence éventuelle de MART-1 a ensuite été révélée à l'aide d'anticorps spécifique anti-MART-1 (S. Rosenberg, NCl, Bethesda, U.S.A.). Les texosomes sécrétés par la lignée tumorale FON (mélanome du patient FON provenant de F. Faure, Institut Pasteur, Paris, France) contiennent l'antigène tumoral MART-1. Des expériences de protection à la protéinase K (Sigma) ont montré que l'épitope de MART-1 reconnu par cet anticorps monoclonal est à l'intérieur des texosomes (résultats non montrés).

Cette première partie du travail montre que :
- les cellules tumorales produisent et sécrètent des vésicules,
- que ces vésicules sont des vésicules d'origine endosomale comportant une membrane externe où se trouvent différentes molécules membranaires (classe I du CMH, différents marqueurs des endosomes),
- que ces vésicules contiennent une fraction de cytosol, y compris des antigènes tumoraux cytosoliques, comme MART-1.

Ces résultats ont amené à vérifier les activités biologiques suivantes des vésicules, désignées texosomes.

### 4. Les texosomes peuvent stimuler des lymphocytes T CD8 in vitro.

Puisque les texosomes portent des molécules de classe I à leur surface, on a testé s'ils sont capables de stimuler des lymphocytes T CD8. On a utilisé deux clones T, LT8 et LT12 donnés par Faure F., Institut Pasteur, Paris, France, reconnaissant un peptide dérivé de MART-1 en association avec HLA-A2 (Dufour et al., 1997). A cette fin, puisque les cellules tumorales FON sont HLA-A2, on a incubé les cellules des clones T LT8 ou LT12 avec des texosomes préparés à partir des sumageants de FON, ou, en guise de contrôle positif, des cellules FON intactes. L'activation des lymphocytes T a été mesurée par la sécrétion de TNFβ. Les texosomes de FON ont induit la sécrétion de TNFβ par LT8 et LT12 d'une façon dose-dépendante (Figure 3). Les cellules FON ont aussi induit une sécrétion de TNFβ, alors que des texosomes dérivés de cellules tumorales n'exprimant pas MART-1 ne le fond pas (figure 3).

Donc, des complexes HLA-A2/peptide de MART-1 sont présents à la surface des texosomes.

Des résultats similaires ont été obtenus chez la souris avec des lymphocytes T de rate d'une souris immunisée par la β-galactosidase (β-gal).

Des texosomes ont été produits à partir de sumageants de cellules de mastocytome P815 ou de cellules P815 exprimant la β-gal (lignées de A. Albina, Institut Gustave Roussy, Villejuif, France), ou de cellules d'une autre tumeur (L1210) leucémie murine H2α) n'exprimant pas la β-gal, L210. Des concentrations croissantes (0,3 µg/ml à 20 *g/ml) de ces différentes préparations de texosomes ont ensuite été incubées pendant 4 jours avec des cellules spléniques de souris immunisées par la β-gal exprimée dans un adénovirus recombinant. Seuls les texosomes de cellules P815 (à la plus forte concentration de 20 µg/mt) exprimant la β-gal ont induit une prolifération significative (voir figure 4) (mesurée par l'incorporation de thymidine tritiée), bien que peu forte, des cellules spléniques. Ces résultats montrent que les texosomes produits par les cellules P815 exprimant la β-gal portent à leur surface des complexes H2^{α}/peptides dérivés de β-gal et sont capables d'activer des lymphocytes T murins.

### 5. Les texosomes peuvent délivrer des antigènes cytosoliques qu'ils contiennent à des cellules présentatrices d'antigènes pour être présentés aux lymphocytes T.

Dans ce but, on a utilisé les clones T LT8 et LT12 reconnaissant spécifiquement un peptide dérivé de MART-1 (MART-1₂₇₋₂₅ = AAGIGILTV, Dufour E. Et al., Diversity of the cytotoxic melanoma-specific immune response. J. Immunol. 1997, 158:3787-3795) en association avec HLA-A2. On a montré que les texosomes produits par la lignée de mélanome humain FON (qui est HLA-A2) contiennent l'antigène tumoral MART-1 (voir figure 2) et sont capables d'activer directement les clones LT8 et LT12. Afin de disposer de texosomes contenant aussi MART-1, mais incapables de stimuler directement les clones LT12 et LT8, on a utilisé la lignée de mélanome MZ2 (T. Boon, Ludwig lnstitute, Bruxelles, Belgique), MART-1 positive mais exprimant un autre élément de restriction que HLA-A2 (HLA-A1 en l'occurrence). En effet, les cellules MZ2, ainsi que les texosomes dérivés de ces cellules, n'activent pas les clones LT8 et LT12 (résultats non-montrés), contrairement aux cellules FON et aux texosomes dérivés de ces cellules (Figure 3). Par contre, quand ces mêmes texosomes dérivés de MZ2 sont incubés en présence de cellules dendritiques exprimant HLA-A2, une stimulation des clones T LT12 et LT8 est observée, comme dans le cas des texosomes dérivés de FON (figure 5).

Dans le cas des texosomes dérivés de MZ2, l'activation des clones T ne peut pas être due à des complexes HLA-A2/peptide dérivés de MART-1 préexistants, puisque elles n'expriment pas l'élément de restriction adéquat (HLA-A2). Par conséquent, il ne peut s'agir que de l'antigène contenu dans les texosomes qui a été repris par les cellules présentatrices d'antigènes, dégradé en peptides qui se sont ensuite associés aux molécules HLA-A2 de la cellule présentatrice. Les texosomes permettent donc le transfert d'un antigène entre une cellule tumorale et une cellule présentatrice d'antigènes. Les texosomes ont donc une fonction semblable à celle des "liposomes naturels".

### 6. Les texosomes induisent la régression de tumeurs solides établies in. vivo.

Enfin, puisque les texosomes sont capables de stimuler des lymphocytes T *in vitro* et de sensibiliser des cellules dendritiques pour l'activation de lymphocytes T spécifiques de tumeurs, on a testé l'activité anti-tumorale des texosomes in vivo.

Pour analyser une telle activité anti-tumorale, on a injecté des souris avec deux fois (10⁵) la dose tumorigène minimale de cellules tumorales d'une tumeur mammaire (cellules TS/A d'haplotype H2^{d}, syngéniques de cellules BALB/c) dans le flanc. Après 3 ou 4 jours, les animaux ayant des tumeurs établies ont été injectés deux fois (jour 3 et 4) avec des texosomes préparés à partir de surnageants de cellules TS/A ou comme contrôle négatif des texosomes de cellules MC38 (S. Rosenberg, NCI, Bethesda, U.S.A.) (cellule tumorale. d'haplotype H2^{b}) ou un volume similaire de PBS. La taille moyenne des tumeurs dans le groupe de souris inoculées avec des préparations de texosomes de TS/A est très diminuée en comparaison avec les groupes de souris contrôle. Cet effet anti-tumoral est dépendant des cellules T, car des souris Nude (souris mutantes dépourvues de lymphocytes T) portant la tumeur et inoculées de façon similaire avec des préparations de texosomes, ne montrent pas de diminution de la masse tumorale (Figure 6B).

Dans cette même série d'expériences, on a observé un effet anti-tumoral des texosomes préparés à partir de cellules de mastocytome P815 d'haplotype H2^{d} ce qui permet de penser que ces deux tumeurs expriment des antigènes communs. Des résultats similaires ont été obtenus avec un autre modèle tumoral très immunogénique, le mastocytome P815 (syngénique de souris DBA/2 et d'haplotype H2^{d}). Dans ce modèle, on a montré que des texosomes injectés en intradermique dans le flanc d'une souris portant une tumeurs (P815) établie au 10ème jour (tumeur mesurant 80-100 mm²) ont capacité de mener à l'éradication de la tumeur dans plus de 60% des cas. De plus, ces souris montrent une immunité anti-tumorale à long terme (résultats non montrés). Dans cette série d'expériences, on a observé des effets anti-tumoraux de texosomes préparés à partir de lymphocytes L1210 isolés à partir d'une leucémie murine d'haplotype H2^{d} et de cellules TS/A, ce qui indique que des épitopes communs existent également entre ces trois tumeurs (entre P815 et TS/A, p53 mutée est commune aux deux tumeurs).

Deux mécanismes pourraient être à la base de l'effet anti-tumoral des texosomes.

Tout d'abord, une fois injectés, les texosomes pourraient activer directement les lymphocytes T de l'hôte, spécifiques de la tumeur. De cette façon, une expansion clonale de lymphocytes T spécifiques de la tumeur ou un "priming" de cellules T pourrait avoir lieu. Une deuxième hypothèse implique une interaction directe des texosomes injectés avec les cellules dendritiques de l'hôte. Ceci pourrait alors stimuler la réponse anti-tumorale. Afin de tester cette deuxième hypothèse, on a suivi la croissance de tumeurs dans des souris injectées en intraveineuse avec des cellules dendritiques dérivées de la moelle osseuse chargées avec des texosomes de cellules tumorales. Des souris DBA/2 (IFFA CREDO, Orléans. France) ont ainsi été injectées avec deux fois (50 x 10⁵) la dose tumorigène minimale de cellules de mastocytome P815. Dix jours après, chaque animal a été injecté avec 5 x 10⁵ cellules dendritiques chargées, et de ce fait sensibilisées, avec 9 µg de texosomes. La taille moyenne des tumeurs dans ces conditions diminue de façon significative en comparaison avec des groupes de souris injectées avec du PBS ou avec des cellules dendritiques chargées de texosomes de cellules d'une tumeur contrôle MC38. En effet, plus de 60% des animaux traités ne présentent plus de tumeur à la fin de l'expérience. De plus, des récidives n'ont pas été observées à long terme (dans 80 % à 100 % des cas). Il est intéressant de constater qu'une telle, dose sous-optimale de texosomes injectée en intradermique n'a pas d'effet, ce qui suggère que les cellules dendritiques peuvent préparer des dexosomes contenant les antigènes tumoraux de façon beaucoup plus efficace que les cellules dendritiques du derme ou les cellules de Langerhans. On a obtenu des résultats similaires avec le modèle de cellules de la tumeur du sein TS/A.

Les résultats obtenus montrent que les texosomes sensibilisent les cellules dendritiques de façon efficace. Ces cellules, ainsi sensibilisées, ont la capacité d'induire des réponses anti-tumorales puissantes *in vivo,* et cela dans le cas de différentes tumeurs. Ces résultats suggèrent que les effets anti-tumoraux observés après l'inoculation directe de texosomes *in vivo* sont dus à la sensibilisation des cellules dendritiques de l'hôte. D'une façon remarquable, l'effet est observé après une seule injection de cellules dendritiques sensibilisées. La majorité des souris traitées montrent une régression tumorale complète ou une survie prolongée (de 60 jours contre 20 jours dans le contrôle) due à la régression tumorale.

La méthode de sensibilisation des cellules présentatrices possède différents avantages par rapport aux méthodes de l'art antérieur :
i) elle ne nécessite pas la connaissance préalable d'antigènes tumoraux : ceci est particulièrement important puisque des antigènes tumoraux ne sont pas connus dans la très grande majorité des tumeurs ;
ii) le procédé peut être appliqué à n'importe quelle tumeur produisant des texosomes ; sur plus de 15 lignées de cellules tumorales testées à ce jour, une seule ne produit pas de texosomes (une des six lignées de mélanome testées).
iii) cette méthode ne dépend pas de l'haplotype du CMH du patient et des cellules tumorales, puisque les antigènes tumoraux présents dans les texosomes sont re-apprêtés et présentés aux lymphocytes T par des molécules du CMH des cellules présentatrices d'antigènes du patient;
iv) le procédé peut en principe être efficace entre tumeurs d'origines différentes, puisqu'il existe des antigènes tumoraux communs non seulement à un type particulier de tumeur (MART-A par exemple dans le mélanome), mais aussi des antigènes communs à des tumeurs complètement différentes (comme les molécules impliquées dans la tumorigenèse, p53 par exemple) ;
v) l'utilisation de texosomes peut aussi se révéler efficace dans le traitement de tumeurs exprimant de faibles niveaux de molécules de classe I du CMH, ou n'en exprimant pas du tout (ces tumeurs représentent 40-50% des cancers métastatiques humains). En effet, les texosomes permettent le transfert d'antigènes intacts entre cellules tumorales et cellules dendritiques, ces antigènes étant ensuite présentés aux lymphocytes T par les molécules de CMH de la cellule dendritique. Les résultats préliminaires montrent que les texosomes permettent d'induire le rejet de tumeurs murines exprimant de faibles niveaux de molécules de classe I du CMH (comme MCA101, S. Rosenberg, NCl, Bethesda, U.S.A.). Ceci est probablement du au fait que les niveaux d'expression de molécules du CMH nécessaires à l'induction d'une réponse immunitaire efficace sont beaucoup plus élevés que ceux nécessaires lors de la phase effectuée (cytotoxicité cellulaire) ;
vi) les texosomes seuls constituent en eux-mêmes, par leur pouvoir immunogène une modalité de vaccination prophylactique ou thérapeutique nouvelle et efficace.

### 7. Les cellules dendritiques produisent des vésicules membranaires immunogènes (Dexosomes)

Cet exemple démontre que les cellules dendritiques produisent des vésicules membranaires et que ces vésicules constituent des vésicules immunogènes puissantes pour l'immunisation antitumorale. Ces vésicules sont particulièrement avantageuses puisqu'elles permettent d'éviter l'étape d'injection *in vivo* des cellules dendritiques entières dont elles proviennent.

La thérapie cellulaire dendritique n'offre pas la certitude du phénotype stable de la cellule injectée, ni de l'homogénéité des compositions cellulaires utilisées. En administrant un produit stable sécrété par ces cellules, c'est-à-dire les vésicules membranaires sus-décrites, on offre à l'hôte porteur de tumeurs une garantie d'efficacité et de pouvoir immunisant.

Dans cet exemple, des cellules dendritiques dérivées de moelle osseuse par traitement pendant 5 jours en GM-CSF+IL-4 (Mayordomo et al., 1995) ont été incubées pendant 3 heures avec des exosomes de cellules tumorales. Les cellules ainsi sensibilisées ont ensuite été cultivées en milieu acide pendant 18 heures (afin de stimuler la production de vésicules), puis des vésicules ont été observées et récoltées selon la méthodologie décrite dans l'exemple 1. Afin de déterminer leur pouvoir immunogène, ces vésicules ont ensuite été incubées in vitro avec des lymphocytes T cytotoxiques spécifiques de l'antigène MART1. Les résultats présentés sur la figure 9 montrent que 0,6 µg de ces vésicules membranaires, appelées DexTexFON (c'est-à-dire dexosome provenant de cellules dendritiques incubées avec des texosomes provenant de cellules tumorales FON), permettent de stimuler directement la prolifération et la sécrétion d'IFNγ de clones LT8 spécifiques de MART-1 et c'est ce que ne peut pas faire 0,6 µg de dexosomes provenant de cellules dendritiques incubées avec des texosomes de cellules tumorales NUN : DexTexNUN (NUN étant une tumeur du rein, HLAA2 négative, MART-1 négative). Ces résultats montrent donc (i) que les cellules dendritiques produisent des vésicules membranaires et que ces vésicules membranaires constituent un immunogène puissant.

En outre, les résultats présentés sur la Figure 9C montrent que, de manière inattendue, la production de dexosomes pour les cellules dendritiques est un phénomène régulé, qui peut être stimulé en présence de certaines cytokines telles IFN-_{γ} et l'IL-10. Ainsi, les résultats présentés montrent que l'IFN-γ ou l'IL-10 augmentent de manière significative (facteur 5 environ), la production de dexosomes. Des résultats comparables ont été observés avec l'IL-12.

### 8. Caractérisation des vésicules membranaires produites par les cellules dendritiques,

La capacité des cellules dendritiques à produire de vésicules membranaires a tout d'abord été confirmée sur des cellules dendritiques produites à partir de précurseurs monocytes humains et sur la lignée de cellules dendritiques murines D1.

La lignée cellulaire D1 et les conditions de maturation de cette lignée ont été décrites par Winzler et al. (J. Exp. Med. 185 (1997) 317).

Les cellules dendritiques dérivées de précurseurs monocytes humains ont été obtenues à partir de la fraction adhérente de cellules mononuclées, prélevées à partir de sujets sains, incubées 7-8 jours en milieu AIMV contenant de la L-Glu, des antibiotiques, 1000 Ul/ml de rhGM-CSF et de rhlL-4 (Schering Plough, Kenilworth, NJ, USA). Après 8 jours de culture, les cellules faiblement adhérentes et les cellules en suspension présentent une morphologie typique de cellules dendritiques, expriment des niveaux élevés de molécules CMH I et II, ainsi que CD40 et CD86. La majeure partie de ces cellules est positive pour CD1a et CD11b et négative pour CD2, CD3, CD14, CD19 et CD83.

Les analyses microscopiques de ces cellules ont fait apparaître la présence de vésicules membranaires riches en molécules du CMH I et II. Ces vésicules ont été isolées par centrifugations et analysées en microscopie immuno-electronique. Plus particulièrement, les sumageants de culture des cellules dendritiques ont été récoltés, centrifugés à 300 g pendant 20 minutes puis à 10 000 g pendant 30 minutes à 4°C, pour éliminer les cellules et les débris cellulaires. Les dexosomes ont ensuite été isolés par une centrifugation à 100 000 g pendant 1h à 4°C, suivie d'un lavage en PBS dans les mêmes conditions (centrifugation à 100 000 g pendant 1h à 4°C). La concentration en protéines dans les préparations de dexosomes a été mesurée par la méthode de Bradford (BioRad Protein Assay [BioRad]).

Les résultats obtenus montrent (Figure 10) une population homogène de vésicules ayant un diamètre compris entre 60 et 90 nm environ. Par ailleurs, plus, de 95% des dexosomes sont marqués par des anticorps anti-CD63, anti-CD82, anti-MHC-I et anti-MHC-II.

Ces résultats confirment que les cellules dendritiques produisent des vésicules membranaires présentant des molécules de présentation des antigènes ainsi que des molécules de co-stimulation lymphocytaire.

### 9. Les dexosomes présentent les antigènes dans un contexte MHC-I restreint.

L'une des caractéristiques avantageuses des dexosomes réside dans la présence de molécules du CMH de classe I. Ces molécules sont en effet nécessaires à la génération d'une réponse cellulaire efficace, et notamment à l'activation et à l'expansion de cellules CTL. La capacité des dexosomes à stimuler les lymphocytes CD8+ et le caractère spécifique des lymphocytes obtenus ont donc été testés.

Pour cela, les cellules dendritiques obtenues à partir de précurseurs monocytes humains (de sujets HLA-A2) ont tout d'abord été sensibilisées par "peptide pulsing" à un antigène particulier. Dans ce but, les cellules (2X10⁶ /ml) ont été incubées pendant 3 à 12 heures, soit en présence de 10 µg/ml du peptide antigénique MART-1/MelanA₍₂₇₋₃₅₎, soit en présence de 10 µg/ml de peptide gp100₍₂₈₀₋₂₈₈₎ (contrôle) en suspension dans l'acide citrique à pH 3,7. Après cette étape de sensibilisation, les dexosomes ont été isolés comme décrit dans l'exemple 1. Les cellules du clone LT12 (clone de CTL HLA-A2 restreint, MART-1 (27-35) spécifique) ont alors été incubées (100 000 CTL par puits) avec des doses croissantes de dexosomes ou de peptides gp100 (contrôle) dans des plaques 96 puits pendant 5 jours. La sécrétion d'interféron gamma par les cellules a ensuite été mesurée par ELISA (Genzyme).

Comme montré sur la Figure 11, les dexosomes portant le peptide MART-1 sont capables de stimuler la production d'interféron gamma par le clone LT12, de manière dose-dépendante. Au contraire, les dexosomes produits à partir des cellules dendritiques pulsées avec le peptide contrôle gp100 n'exercent aucun effet stimulant sur ce clone.

Ces résultats confirment que les molécules du CMH-I exprimées par les dexosomes de l'invention sont fonctionnelles.

### 10. Les dexosomes bloquent la croissance tumorale in vivo.

Cet exemple démontre la capacité des dexosomes à induire une réponse immunitaire in vivo et plus particulièrement à induire la prolifération de cellules T spécifiques d'une tumeur.

Des cellules dendritiques obtenues à partir de moelle osseuse ont été chargées avec un éluat acide de tumeur comportant différents- peptides antigéniques tumoraux. La technologie de préparation et de sensibilisation des cellules dendritiques a été décrite par Zitvogel et al (1996). La figure 12 présente les marqueurs exprimés par les dexosomes produits par ces cellules dendritiques. Comme indiqué précédemment, cette figure montre la présence abondante de molécules du CMH de classe I et de classe II ainsi que les marqueurs CD86 et le récepteur à la transferrine. Au contraire, bien qu'ils apparaissent dans les lysats cellulaires, les marqueurs H2-M, li et calnexine sont indétectables dans les préparations exosomales.

Deux modèles expérimentaux de tumeurs ont été choisis pour tester les propriétés anti-tumorales in vivo des dexosomes de l'invention. Le premier modèle, P815, est un mastocytome agressif syngénique de DBA/2 (H2^{d}) pour lequel très peu d'immunothérapies efficaces ont été rapportées sur des tumeurs établies au jour 10. Le modèle TS-A est un carcimome mammaire spontané faiblement immunogène exprimant des niveaux plus faibles de molécules du CMH de classe l, syngénique de BALB/c (H2^{d}). Les peptides tumoraux des tumeurs P815 ou TS/A élués par traitement acide ont été chargés sur les cellules syngéniques dendritiques, dérivées de la moelle osseuse, comme décrit précédemment. Les dexosomes ont ensuite été préparés à partir des surnageants de ces cellules dendritiques et utilisées pour l'immunisation in vivo.

### Souris et lignées cellulaires tumorales.

Les souris femelles DBA/2J (H2^{d}) et BALB/c (H2^{d}) âgées de six à huit semaines ont été achetées au Laboratoire Iffa Credo, Lyon, France et maintenues dans des conditions dépourvues de pathogène. Les souris nude ont été maintenues dans un micro-environnement protégé. Les cellules P815 ont été fournies par T. Boon (Ludwig Institute, Belgique). Le modèle TS/A a été fourni par Guido Fomi (Immunogenetic and Histocompatibility Center, Turin, Italie). Toutes les lignées tumorales ont été conservées dans du milieu RPMI 1640 supplémenté par 10% de sérum de veau foetal dépourvu d'endotoxine (Gibco BRL), 2 mM de L-Glutamine, 100 Unités/ml de pénicilline, 100 mg/ml de streptomycine, acides aminés essentiels et pyruvate. Ce milieu est également désigné dans ce qui suit : milieu CM.

### Protocole et résultats.

Deux fois la dose tumorigène minimale de cellules tumorales (5x10⁵ P815, 10⁵ TS/A) ont été inoculées en intradermique dans la zone supérieure du flanc droit des souris DBA/2 et BALB/c respectivement. Les animaux présentant des tumeurs TS/A établies de trois à quatre jours ou des tumeurs P815 établies de six à dix jours ont ensuite été immunisés par une injection intradermique unique de 3 à 5 µg de dexosomes dans la partie inférieure du flan ipsilatéral. Ces procédures ont été effectuées de manière similaire à la fois dans les animaux immunocompétents et dans les souris nude. Une seule injection thérapeutique a été réalisée pour chaque souris. La taille des tumeurs a été contrôlée deux fois par semaine et les souris ont été sacrifiées lorsqu'elles, portaient des tumeurs ulcérées ou d'une taille trop importante. Chaque série d'expérience a été réalisée deux à trois fois en utilisant des groupes de cinq souris pour chaque traitement individuel. Les résultats obtenus sont présentés sur la figure 13. Comme le montre la figure 13B, le traitement de tumeurs P815 établies au jour 10 (ayant une taille de 50 à 90 mm²) a pu être réalisé par une seule administration intradermique de 3 à 5 µg de dexosomes par souris. En une semaine, la croissance tumorale s'est arrêtée dans les groupes ayant reçu les dexosomes dérivés de cellules dendritiques chargées avec le peptide tumoral autologue, et dans 40 à 60 % des souris, les tumeurs avaient disparu entièrement au jour 60.

Ces animaux présentent par ailleurs une réponse immune durable et ont rejeté une injection supplémentaire de P815 normalement létale pour des souris non traitées. En revanche, ces souris ne sont pas protégées contre une injection du clone leucémique syngénique L1210, ce qui montre bien le caractère immunospécifique de l'effet obtenu. Enfin, les groupes de souris immunisées avec des dexosomes contrôles (chargées avec des peptides de la rate de la souris) ne présentent aucun effet anti-tumoral de même que les souris non traitées. Ces résultats montrent donc que les dexosomes chargés en peptide tumoraux selon l'invention sont capables d'induire une régression tumorale in vivo.

Des effets anti-tumoraux similaires ont été obtenus sur le modèle de tumeur TS/A comprenant des tumeurs établies aux jours 3/4. Dans cette série d'expériences, toutes les souris présentaient un retard de croissance tumoral statistiquement significatif qui prolongeaient leur survie (figure 14). Cet effet anti-tumoral n'a pas été observé dans les souris athymiques Nu/Nu comme indiqué sur la figure 14B, ce qui montre que la présence de cellules T est nécessaire à l'expression de l'effet anti-tumoral des dexosomes de l'invention.

De plus, l'expérience suivante montre que les dexosomes stimulent directement une réponse CTL spécifique dans les animaux présentant la tumeur P815. Les splénocytes de souris ayant rejeté les tumeurs P815 après immunisation avec des dexosomes ont été récoltés au jour 90 et cultivés pendant cinq jours en présence de cellules P815 irradiées exprimant l'antigène B7.1 pour augmenter la fréquence de précurseurs spécifiques. Ces cellules effectrices ont été testées dans un essai de libération du chrome contre (i) les cellules tumorales autologues P815 (H2^{d}), (ii) les cellules non apparentées L1210 et (ii) les cellules YAC. Une activité cytolytique spécifique significative a été observée contre les cellules P815 dans les splénocytes de souris immunisées avec les dexosomes (figure 15). De manière intéressante, aucune des rates de souris rejetant spontanément la tumeur P815 ou présentant des tumeurs P815 ne présentent cette activité cytolytique dans les mêmes conditions. Ces résultats montrent qu'une injection unique de dexosomes selon l'invention dérivés de cellules dendritiques sensibilisées avec un antigène ou des peptides antigéniques correspondants, est capable de déclencher de manière efficace une réponse spécifique anti-tumorale CTL *in vivo.*

Pour déterminer si la réponse immunitaire et anti-tumorale induite par les dexosomes est restreinte aux MHC et pas simplement due à un effet direct des peptides tumoraux, des cellules dendritiques dérivées des souris H2^{d} (DBA/2) ou H2^{b} (C57BL/6) ont été chargées en parallèle avec des peptides tumoraux élués de la tumeur P815. Les dexosomes produits par ces cellules dendritiques de souris ont ensuite été isolés et utilisés séparément pour des injections intradermiques directes dans les souris DBA/2 portant des tumeurs P815 établies au jour 6/10.

Comme le montre la figure 138, seuls les dexosomes portant les peptides tumoraux syngéniques sont des vaccins anti-tumoraux efficaces (induisant dans 60% des souris une disparition de la tumeur) alors que les dexosomes des cellules dendritiques allogéniques n'induisent pratiquement aucun effet anti-tumoral. Ces résultats indiquent que les dexosomes selon l'invention induisent une réponse anti-tumorale *in vivo* restreinte au MHC.

Des expériences similaires à celles décrites ci-dessus ont été réalisées en procédant à des injections non pas intradermiques mais intraveineuses. Les résultats obtenus sont présentés sur la figure 16. Ils montrent tout d'abord une régression tumorale consécutivement à l'injection intraveineuse de dexosomes. De plus, ces résultats montrent que les dexosomes sont plus puissants que les cellules dendritiques dont ils dérivent pour éradiquer les tumeurs in vivo. Ces résultats illustrent donc les propriétés remarquables et inattendues des dexosomes.

Les résultats présentés ci-dessus montrent donc que les cellules dendritiques immatures humaines ou murines sécrètent des dexosomes, que ces dexosomes présentent des molécules non seulement du CMH de classe II mais également du CMH de classe I ainsi que des molécules co-stimulantes, et enfin que ces dexosomes sont immunogènes et induisent une régression tumorale *in vivo.*

Les dexosomes peuvent être obtenus dans des quantités relativement élevées (1µg par million de cellules dendritiques par dix-huit heures, selon le test Bradford) à partir du milieu de cultures de cellules dendritiques (cellules dendritiques dérivées de moelle osseuse en présence de GM-CSF + IL4, lignées de cellules dendritiques D1 ou cellules dendritiques dérivées de précurseurs monocytes humains, isolées de cellules mononucléées du sang périphérique). Les dexosomes ont été caractérisés sur le plan morphologique et biochimique. Les vésicules membranaires analysées par microscopie immunoélectronique représentent une population homogène de vésicules ayant un diamètre d'environ 60 à 90 nannomètres. Les préparations dexosomales sont apparemment dépourvues de rétrovirus, membranes plasmiques, constituant microsomaux ou corps apoptotiques. Les dexosomes surexpriment de manière abondante les molécules du CMH de classe II, I, CD63 et CD86 comparativement aux membranes plasmiques. Aucun compartiment dérivé du réticulum endoplasmique n'a été détecté dans les dexosomes par Western Blotting, en utilisant des anticorps anti-calnexine. Une mort cellulaire programmée n'a pas pu être mise en évidence dans ces cultures en utilisant différentes conditions. De manière intéressante, la production de ces vésicules apparaît comme un phénomène régulé. La quantité de vésicules semblent pouvoir être diminuée en induisant une maturation des cellules dendritiques, comme déterminé par le test Bradford, Western Blotting, et microscopie immunoélectronique. De plus, le niveau de sécrétion de ces vésicules peut être amélioré de manière significative en diminuant le pH du milieu de culture, ou en incubant les cellules en présence de certaines cytokines, ou encore en traitant les cellules dendritiques par irradiation. Ce phénomène est particulièrement inattendu dans la mesure où les cellules dendritiques immatures sont généralement considérées comme ayant un faible pouvoir de présentation d'antigène et donc une faible activité immunologique. Les résultats présentés montrent que ce sont les cellules dendritiques à ce stade immatures qui possèdent la propriété de produire des dexosomes de manière efficace. Les résultats présentés montrent enfin que ces dexosomes sont capables de déclencher une réponse par des cellules T efficaces à la fois *in vitro* et *in vivo* et qu'elles sont également capables d'induire la régression de tumeur *in vivo.* Ces vésicules constituent donc bien des candidats particulièrement attractifs pour des approches immunothérapies en système non-cellulaire.

### 11. Purification d'exosomes par électrophorèse en phase fluide

Cet exemple décrit l'utitisation d'une méthode originale de purification d'exosomes basée sur l'électrophorèse en phase fluide.

L'électrophorèse en phase fluide est une méthode préparative de séparation de composés biologiques d'après leur charge. Cette méthode a été utilisée pour séparer des protéines par isoélectrofocalisation. Cette méthode peut présenter les avantages suivants :
- c'est une méthode préparative permettant une injection continue de matériel, et donc la purification de grandes quantités de vésicules.
- cette méthode permet la purification des dexosomes en une ou deux étapes, éliminant potentiellement les étapes de centrifugation.

Afin de déterminer si cette méthode est applicable à la purification d'exosomes, nous avons réalisé l'expérience suivante:

Une préparation de dexosomes isolés à partir d'un surnageant de cellules dendritiques murines par ultracentrifugation différentielle a été injectée dans l'électrophorèse en phase fluide, dans les conditions habituelles décrites par Amigorena et al. (Nature, 369 (1994), 113). 40 fractions ont été récoltées et la concentration protéique dans chacune de ces fractions a été déterminée par le test de Bradford (BioRad), afin de détecter la présence des dexosomes. Comme le montre la figure 17, 90% des dexosomes ont été trouvés concentrés dans quatre fractions d'EPF. La migration des dexosomes dans un pic étroit selon cette technologie démontre la faisabilité de l'électrophorèse comme méthode d'isolement des dexosomes.

### Références

Amigorena, S., Drake, J. R., Webster, P., and Mellman. I. (1994). Transient accumulation of new class Il molecules in a novel endocytic compartment in B lymphocytes [see comments]. Nature *369*, 113-120.

Boczkowshi. D., Nair, S. K., Snyder, D., and Gilboa, E. (1996). Dendritic cells pulsed with RNA are potent antigen-presenting cells *in vitro* and *in vivo.* Journal of Experimental Medicine *184*, 465-72.

Boon, T. (1992). Toward a genetic analysis of tumor rejection antigens. Advances in Cancer Research *58,* 177-210.

Espevik, T. & Nissen-Meyer. J. (1986) J. Immunol. Methods *95*:99-103.

Felder, S., Miller, K., Moehren, G., Ullrich, A., Schlessinger, J., and Hopkins, C. R. (1990). Kinase activity controls the sorting of the epidermal growth factor receptor within the multivesicular body. Cell *61*, 623-634.

Mayordomo, J. I., Zorina, T., Stork-us, W. J., Zitvogel, L., Celluzzi, C., Falo, L. D., Melief, C. J., Ildstad, S. T., Kast, W. M., Deleo, A. B., and et al. (1995). Bone marrow-derived dendritic cells pulsed with synthetic tumour peptides elicit protective and therapeutic antitumour immunity. Nature Medicine *1*, 1297-302.

Nabel, G.J., Gordon, D., Bishop, D.K., Nicholoff, B.J., Yang, Z.Y., Avuga, A., Cameron, M.J., Nabel, E.G., Chang, A.E. (1996) Immune response in human melanoma after transfer of an allogenic class I major histocompatibility complex gene with DNA-liposome complexes. Proc. Natl. Acad. Sci. USA 93:15388-18393.

Pardoll, D. M. (1995). Paracrine cytokine adjuvants in cancer immunotherapy. Annual Review of Immunology *13*, 399-415.

Raposo, G., Nijman, H. W., Stoorvogel, W., Leijendekker, R., Harding, C. V., Melief, C. J. M., and Gcuzc, H. J. (1996). B lymphocytes secrète antigen-presenting vesicles. J. Exp. Med. *183*, 1161-1172,

Rosenberg, S. A., Kawakami, Y., Robbins. P. F., and Wang. R. (1996). Identification of the genes encoding cancer antigens: implications for cancer immunotherapy. Advances in Cancer Research *70*, 145-77.

Traversasi. C., Van der Bruggen, P., Leuscher, I.F.. Lurguin, C., Chamez, P., Van Del, A., De Places. E., Amar-Costesec, A. and Boon, T. (1992) A nonapeptide encoded by human gene MART-1 is recognized on HLA-AI by cytotoxic T lymphocytes directed against tumor antigen MZ2-E. J. Exp. Med. 176: 1453-1457.

Walker S.A. *et al.* (1997). Nature, vol. 387, pp. 61 et suivantes.

Zitvogel, L., Robbins, P. D., Storkus, W. J., Clarke, M. R., Maeurer, M. J., Campbell, R. L., Davis, C. G., Tahara, H., Schreiber, R. D., and Lotze, M. T. (1996). Interleukin-12 and B7.1 co-stimulation cooperate in the induction of effective antitumor immunity and therapy of established tumors. European Journal of Immunology *26*, 1335-41 **[a].**

Zitvogel, L., Mayordomo, J. I., Tjandrawan, T., DeLeo, A. B., Clarke, M. R., Lotze, M. T., and Storkus, W. J. (1996). Therapy of murine tumors with tumor peptide-pulsed dendritic cells: dependence on T cells, B7 costimulation. and T helper cell 1-associated cytokines [see comment]. Journal of Experimental Medicine *183*, 87-97 **[b]**.

**Tableau 1.**

| **Production de texosomes par des lignées de cellules tumorales murines et humaines.** | |
|---|---|
| LIGNÉES DE CELLULES TUMORALES | TEXOSOMES (µG/2×10⁷ CELLULES/18 H) |
| Murines | |
| MCA101 | 172 |
| P815 | 163 |
| MC38 | 120 |
| L1210 | 150 |
| TS/A | 160 |
| Humaines (mélanomes) | |
| VIO* | 80 |
| FON | 90 |
| MZ2-2 | 18 |
| Humaines (néphromes) | |
| RCC NUN* | 120 |
| RCC JOUA* | 18 |
| RCC MEG* | 10 |
| RCC GIAM* | 100 |

## Revendications

1. Vésicule membranaire débarrassée de son environnement naturel, **caractérisée en ce qu'**elle est sécrétée par une cellule dendritique et **en ce qu'**elle comporte des molécules du complexe majeur d'histocompatibilité de classe I et des molécules du complexe majeur d'histocompatibilité de classe II.

2. Vésicule membranaire selon la revendication 1, **caractérisée en ce qu'**elle est sécrétée par une cellule dendritique sensibilisée à un antigène.

3. Vésicule membranaire selon la revendication 1, **caractérisée en ce qu'**elle comporte un ou plusieurs peptides antigéniques.

4. Vésicule membranaire désignée dexosome, selon l'une des revendications 1 à 3, **caractérisée en ce qu'**elle dérive d'une cellule dendritique humaine.

5. Vésicule selon la revendication 4, **caractérisée en ce qu'**elle comporte une ou plusieurs molécules CD63.

6. Vésicule selon la revendication 4 ou 5, **caractérisée en ce qu'**elle comporte une ou plusieurs molécules CD82 et/ou CD86.

7. Vésicule selon l'une des revendications 4 à 6, **caractérisée par** un diamètre compris entre 60 et 90 nm.

8. Vésicule selon l'une des revendications 4 à 7, **caractérisée en ce qu'**elle dérive d'une cellule dendritique immature.

9. Vésicule selon l'une des revendications 4 à 8, **caractérisée en ce qu'**elle dérive d'une cellule dendritique porteuse d'un ou plusieurs peptides antigéniques.

10. Vésicule selon la revendication 9, **caractérisée en ce qu'**elle dérive d'une cellule dendritique incubée avec une vésicule dérivée d'une cellule tumorale, débarrassée de son environnement naturel, comprenant une bicouche lipidique qui entoure une fraction cytosolique, ladite vésicule tumorale présentant sur sa surface, des molécule de classe I du complexe majeur d'histocompatibilité ou CMH, dans des conditions permettant la sensibilisation de la cellule dendritique avec les vésicules d'origine tumorale, et la récupération d'une vésicule présentant des molécules de classe I du CMH et des molécules de classe II du CMH.

11. Vésicule selon la revendication 9 **caractérisée en ce qu'**elle dérive d'une cellule dendritique immortalisée.

12. Procédé de préparation in-vitro d'une vésicule selon l'une des revendications 1 à 11, comprenant une première étape d'obtention de cellules dendritiques ou de culture cellulaire comprenant des cellules dendritiques, une deuxième étape de sensibilisation des cellules dendritiques à un antigène ou à un groupe d'antigènes, et une troisième étape comprenant la récupération de vésicules produites par les cellules dendritiques à partir de ces cultures cellulaires.

13. Procédé selon la revendication 12, **caractérisé en ce que** la première étape comprend l'obtention de cellules dendritiques à partir de précurseurs monocytes ou de moelle osseuse.

14. Procédé selon la revendication 12 ou 13, **caractérisé en ce que** la première étape comprend l'obtention de cellules dendritiques immatures.

15. Procédé selon la revendication 14, **caractérisé en ce que** les cellules dendritiques immatures sont humaines.

16. Procédé selon l'une des revendications 12 à 15, **caractérisé en ce que** les cellules dendritiques sont sensibilisées par mise en contact avec des peptides, des antigènes, des cellules ou membranes ou vésicules exprimant des antigènes ou des peptides antigéniques, des liposomes ou des acides nucléiques.

17. Procédé selon la revendication 16, **caractérisé en ce que** les acides nudéiques sont incorporés dans des vecteurs chimiques ou viraux.

18. Procédé selon l'une des revendications 12 à 17, **caractérisé en ce qu'**il comporte une première étape, facultative, de traitement des cellules.

19. Procédé selon la revendication 18, **caractérisé en ce que** les cellules dendritiques sont traitées par culture en présence de cytokines favorisant l'état immature, par irradiation, ou en diminuant le pH de la culture, ou en combinant ces différents types de traitement

20. Procédé selon la revendication 12, **caractérisé en ce que** l'isolement des vésicules est réalisé par centrifugation, électrophorèse, chromatographie et/ou nanofiltration.

21. Utilisation de vésicules selon l'une des revendications 1 à 11, pour la stimulation in vitro de lymphocytes T spécifiques d'antigènes contenus dans les susdites vésicules ou de lymphocytes B.

22. Utilisation selon la revendication 21, **caractérisée en ce que** lesdites vésicules sont utilisées pour la stimulation et l'amplification in vitro de lymphocytes T spécifiques d'antigènes contenus dans lesdites vésicules.

23. Utilisation de vésicules selon l'une des revendications 1 à 11, pour la sélection *ex vivo* d'un répertoire de lymphocytes T, susceptibles de reconnaître des antigènes spécifiques contenus dans les susdites vésicules.

24. Médicament comprenant à titre de substance active une ou plusieurs vésicules selon l'une des revendications 1 à 11, en association avec un véhicule pharmaceutiquement acceptable.

25. Médicament selon la revendication 24, pour le traitement des cancers, des maladies infectieuses ou des maladies parasitaires.

26. Médicament selon la revendication 24 ou 25, **caractérisé en ce qu'**il comprend en outre un agent stabilisant.

27. Médicament selon l'une des revendications 25 à 26, **caractérisé en ce qu'**il comprend un adjuvant immunostimulant.

28. Médicament selon la revendication 25, pour le traitement d'une tumeur solide.

29. Médicament selon la revendication 28, **caractérisé en ce que** la tumeur solide est choisie parmi le cancer du rein, du sein, du colon, du poumon, de l'estomac, du foie, le mélanome et le sarcome.

30. Médicament selon la revendication 25, pour le traitement d'une tumeur hématopoïétique.

31. Médicament selon la revendication 30, **caractérisé en ce que** la tumeur hématopoïétique est choisie parmi les leucémies et les lymphomes malins hodgkiniens ou non hodgkiniens.

32. Utilisation d'une vésicule selon l'une des revendications 1 à 11, pour la préparation d'une composition pharmaceutique destinée au traitement des cancers, des maladies infectieuses ou des maladies parasitaires.

33. Utilisation de l'interféron gamma, de l'interleukine 10 et/ou de l'interleukine 12 pour la production en culture de vésicules selon l'une des revendications 1 à 11.

## Patentansprüche

1. Membranvesikel, das von seiner natürlichen Umgebung freigesetzt ist, **dadurch gekennzeichnet, dass** es von einer dendritischen Zelle sezerniert ist und dass es Moleküle des Klasse I Haupthistokompatibilitätskomplexes und Moleküle des Klasse II Haupthistokompatibilitätskomplexes umfasst.

2. Membranvesikel nach Anspruch 1, **dadurch gekennzeichnet, dass** es von einer gegen ein Antigen sensibilisierten dendritischen Zelle sezerniert ist.

3. Membranvesikel nach Anspruch 1, **dadurch gekennzeichnet, dass** es ein oder mehrere antigene Peptide umfasst.

4. Membranvesikel, als Dexosom bezeichnet, nach einem der Ansprüche 1 bis 3, **dadurch gekennzeichnet, dass** es von einer humanen dendritischen Zelle stammt.

5. Vesikel nach Anspruch 4, **dadurch gekennzeichnet, dass** es ein oder mehrere CD63 Moleküle umfasst.

6. Vesikel nach Anspruch 4 oder 5, **dadurch gekennzeichnet, dass** es ein oder mehrere CD82 und/oder CD86 Moleküle umfasst.

7. Vesikel nach einem der Ansprüche 4 bis 6, das durch einen Durchmesser zwischen 60 und 90 nm gekennzeichnet ist.

8. Vesikel nach einem der Ansprüche 4 bis 7, **dadurch gekennzeichnet, dass** es von einer unreifen dendritischen Zelle stammt.

9. Vesikel nach einem der Ansprüche 4 bis 8, **dadurch gekennzeichnet, dass** es von einer dendritischen Zelle stammt, die ein oder mehrere antigene Peptide trägt.

10. Vesikel nach Anspruch 9, **dadurch gekennzeichnet, dass** es von einer dendritischen Zelle stammt, die mit einem von einer Tumorzelle stammenden Vesikel inkubiert ist, und von seiner natürlichen Umgebung freigesetzt ist, umfassend eine Lipid-Doppelschicht, die eine Cytosolfraktion umschließt, wobei besagtes Tumor-Vesikel auf seiner Oberfläche Moleküle des Klasse I Haupthistokompatibilitätskomplexes oder MHC aufweist, unter Bedingungen, die die Sensibilisierung der dendritischen Zelle mit den Vesikeln Tumorursprungs und die Wiedergewinnung eines Vesikels erlauben, das Moleküle des Klasse I MHC und Moleküle des Klasse II MHC aufweist.

11. Vesikel nach Anspruch 9, **dadurch gekennzeichnet, dass** es von einer immortalisierten dendritischen Zelle stammt.

12. Verfahren zur in vitro Herstellung eines Vesikels nach einem der Ansprüche 1 bis 11, umfassend einen ersten Schritt der Gewinnung dendritischer Zellen oder einer dendritische Zellen umfassenden Zellkultur, einen zweiten Schritt der Sensibilisierung dendritischer Zellen gegen ein Antigen oder eine Gruppe Antigene und einen dritten Schritt, umfassend die Wiedergewinnung der von den dendritischen Zellen gebildeten Vesikel aus diesen Zellkulturen.

13. Verfahren nach Anspruch 12, **dadurch gekennzeichnet, dass** der erste Schritt die Gewinnung dendritischer Zellen aus Vorläufer-Monocyten oder Knochenmark umfasst.

14. Verfahren nach Anspruch 12 oder 13, **dadurch gekennzeichnet, dass** der erste Schritt die Gewinnung unreifer dendritischer Zellen umfasst.

15. Verfahren nach Anspruch 14, **dadurch gekennzeichnet, dass** die unreifen dendritischen Zellen humane Zellen sind.

16. Verfahren nach einem der Ansprüche 12 bis 15, **dadurch gekennzeichnet, dass** die dendritischen Zellen durch Inkontaktbringen mit Peptiden, Antigenen, Zellen oder Membranen oder Vesikeln, die Antigene oder antigene Peptide exprimieren, Liposomen oder Nucleinsäuren sensibilisiert sind.

17. Verfahren nach Anspruch 16, **dadurch gekennzeichnet, dass** die Nucleinsäuren in chemischen oder viralen Vektoren inkorporiert sind.

18. Verfahren nach einem der Ansprüche 12 bis 17, **dadurch gekennzeichnet, dass** es einen fakultativen ersten Schritt der Behandlung von Zellen umfasst.

19. Verfahren nach Anspruch 18, **dadurch gekennzeichnet, dass** die dendritischen Zellen durch Kultivierung vorzugsweise des unreifen Zustandes in Anwesenheit von Cytokinen, durch Bestrahlung oder Verringerung des pH der Kultur oder Kombination dieser verschiedenen Behandlungsarten behandelt sind.

20. Verfahren nach Anspruch 12, **dadurch gekennzeichnet, dass** die Isolierung der Vesikel mittels Zentrifugation, Elektrophorese, Chromatographie und/oder Nanofiltration durchgeführt ist.

21. Verwendung der Vesikel nach einem der Ansprüche 1 bis 11 zur in vitro Stimulation von T-Lymphocyten, die für Antigene spezifisch sind, die in oben genannten Vesikeln oder B-Lymphocyten enthalten sind.

22. Verwendung nach Anspruch 21, **dadurch gekennzeichnet, dass** die besagten Vesikel für die in vitro Stimulation und Zunahme von T-Lymphocyten verwendet sind, die für in besagten Vesikeln enthaltene Antigene spezifisch sind.

23. Verwendung der Vesikel nach einem der Ansprüche 1 bis 11 zur ex vivo Selektion eines Repertoires von T-Lymphocyten, die in der Lage sind. in oben genannten Vesikeln enthaltene spezifische Antigene wieder zu erkennen.

24. Arzneimittel, umfassend als aktive Substanz ein oder mehrere Vesikel nach einem der Ansprüche 1 bis 11 in Kombination mit einem pharmazeutisch verträglichen Träger.

25. Arzneimittel nach Anspruch 24 zur Behandlung von Krebserkrankungen, Infektionskrankheiten oder parasitären Krankheiten.

26. Arzneimittel nach Anspruch 24 oder 25, **dadurch gekennzeichnet, dass** es außerdem ein Stabilisierungsmittel umfasst.

27. Arzneimittel nach einem der Ansprüche 25 bis 26, **dadurch gekennzeichnet, dass** es ein immunstimulierendes Adjuvans umfasst.

28. Arzneimittel nach Anspruch 25 zur Behandlung eines festen Tumors.

29. Arzneimittel nach Anspruch 28, **dadurch gekennzeichnet, dass** der feste Tumor aus Nierenkrebs, Brustkrebs, Darmkrebs, Lungenkrebs, Magenkrebs, Leberkrebs, Melanom und Sarkom ausgewählt ist.

30. Arzneimittel nach Anspruch 25 zur Behandlung eines hämatopoetischen Tumors.

31. Arzneimittel nach Anspruch 30, **dadurch gekennzeichnet, dass** der hämatopoetische Tumor aus Leukämien und malignen Hodgkin- und Nicht-Hodgkin-Lymphomen ausgewählt ist.

32. Verwendung eines Vesikels nach einem der Ansprüche 1 bis 11 zur Herstellung einer pharmazeutischen Zusammensetzung, die zur Behandlung von Krebserkrankungen, Infektionskrankheiten oder parasitären Krankheiten bestimmt ist.

33. Verwendung von gamma-Interferon, Interleukin 10 und/oder interleukin 12 zur Herstellung von Vesikeln nach einem der Ansprüche 1 bis 11 in Kultur.

## Claims

1. A membrane vesicle devoid of its natural environment, wherein said vesicle is secreted by a dendritic cell and comprises Class I molecules of the major histocompatibility complex and Class II molecules of the major histocompatibility complex.

2. Membrane vesicle according to claim 1, **characterized in that** it is secreted by a dendritic cell sensitized to an antigen.

3. Membrane vesicle according to claim 1, **characterized in that** it comprises one or several antigenic peptides.

4. Membrane vesicle designated dexosome, according to any one of claims 1 to 3, wherein said vesicle is derived from a human dendritic cell.

5. Vesicle according to claim 4, **characterized in that** it comprises one or several CD63 molecules.

6. Vesicle according to claim 4 or 5, **characterized in that** it comprises one or several CD82 and/or CD86 molecules.

7. Vesicle according to any one of claims 4 to 6, **characterized by** a diameter comprised between 60 and 90 nm.

8. Vesicle according to any one of claims 4 to 7, **characterized in that** it is derived from an immature dendritic cell.

9. Vesicle according to any one of claims 4 to 8, wherein said vesicle is derived from a dendritic cell carrying one or several antigenic peptides.

10. Vesicle according to claim 9, wherein said vesicle derives from a dendritic cell incubated with a vesicle derived from a tumor cell, devoid of its natural environment, comprising a lipid bilayer that surrounds a cytosolic fraction, said tumoral vesicle presenting, on its surface, Class I molecules of the major histocompatibility complex or MHC, under conditions allowing the sensitization of the dendritic cell with the vesicles of tumor origin, and recovering a vesicle presenting Class I molecules of the MHC and Class II molecules of the MHC.

11. Vesicle according to claim 9, **characterized in that** said vesicle derives from an immortalized dendritic cell.

12. A method for preparing in vitro a membrane vesicle according to any one of claims 1 to 11, comprising a first step of obtaining dendritic cells or a cell culture comprising dendritic cells, a second step of sensitizing the dendritic cells to an antigen or a group of antigens, and a third step comprising recovering vesicles produced by dendritic cells from said cell cultures.

13. The method according to claim 12, **characterized in that** the first step comprises obtaining dendritic cells from monocyte precursors or bone marrow.

14. The method according to claim 12 or 13, **characterized in that** the first step comprises obtaining immature dendritic cells.

15. The method according to claim 14, wherein said immature dendritic cells are human.

16. The method according to any one of claims 12 to 15, **characterized in that** the dendritic cells are sensitized by contacting with peptides, antigens, cells or membranes or vesicles expressing antigens or antigenic peptides, liposomes or nucleic acids.

17. The method of claim 16, **characterized in that** the nucleic acids are incorporated into chemical or viral vectors.

18. The method according to any one of claims 12 to 17, **characterized in that** it comprises a first, optional step of cell treatment.

19. The method according to claim 18, **characterized in that** the dendritic cells are treated by culture in the presence of cytokines favoring the immature state, by irradiation, or by reducing the pH of the culture, or by a combination of these different types of treatment.

20. The method according to claim 12, **characterized in that** the vesicles are isolated by performing a centrifugation, electrophoresis, chromatography and/or nanofiltration.

21. The use of vesicles according to any one of claims 1 to 11, for the in vitro stimulation of T lymphocytes specific for antigens contained in said vesicles, or of B lymphocytes.

22. Use according to claim 21, **characterized in that** said vesicles are used for the in vitro stimulation and amplification of T lymphocytes specific for antigens contained in said vesicles.

23. The use of vesicles according to any one of claims 1 to 11, for the ex vivo selection of a repertoire of T lymphocytes capable of recognizing specific antigens contained in said vesicles.

24. A medicament comprising, as an active substance, one or several vesicles according to any one of claims 1 to 11, in association with a pharmaceutically acceptable vehicle.

25. Medicament according to claim 24, for treating cancers, infectious diseases or parasitic diseases.

26. Medicament according to claim 24 or 25, **characterized in that** it further comprises a stabilizing agent.

27. Medicament according to any one of claims 24 to 26, **characterized in that** it comprises an immunostimulating adjuvant.

28. Medicament according to claim 25, for treating a solid tumor.

29. Medicament according to claim 28, **characterized in that** the solid tumor is selected from kidney, breast, colon, lung, stomach, liver, melanoma and sarcoma cancers.

30. Medicament according to claim 25, for treating a hematopoietic tumor.

31. Medicament according to claim 30, **characterized in that** the hematopoietic tumor is selected from leukemia and hodgkin or non-hodgkin malignant lymphoma.

32. The use of a vesicle according to any one of claims 1 to 11, for the manufacture of a pharmaceutical composition for treating cancers, infectious diseases or parasitic diseases.

33. The use of gamma interferon, interleukin-10 and/or interleukin-12 for the production in culture of vesicles according to any one of claims 1 to 11.
